(19) Europäisches Patentamt / European Patent Office / Office européen des brevets

(11) **EP 2 117 705 B1**

(12) **EUROPÄISCHE PATENTSCHRIFT**

(45) Veröffentlichungstag und Bekanntmachung des Hinweises auf die Patenterteilung:
**26.11.2014 Patentblatt 2014/48**

(21) Anmeldenummer: **08708607.0**

(22) Anmeldetag: **04.02.2008**

(51) Int Cl.:
*B01J 23/96* (2006.01)    *B01J 38/20* (2006.01)
*C07C 5/48* (2006.01)    *C07C 45/33* (2006.01)
*C07C 45/35* (2006.01)    *C07C 51/215* (2006.01)
*C07C 51/25* (2006.01)

(86) Internationale Anmeldenummer:
**PCT/EP2008/051303**

(87) Internationale Veröffentlichungsnummer:
**WO 2008/095877 (14.08.2008 Gazette 2008/33)**

(54) **VERFAHREN ZUR REGENERIERUNG EINES IM RAHMEN EINER HETEROGEN KATALYSIERTEN PARTIELLEN DEHYDRIERUNG EINES KOHLENWASSERSTOFFS DEAKTIVIERTEN KATALYSATORBETTS**

PROCESS FOR REGENERATION OF A CATALYST BED DEACTIVATED IN THE CONTEXT OF A HETEROGENEOUSLY CATALYZED PARTIAL DEHYDROGENATION OF A HYDROCARBON

PROCÉDÉ DE RÉGÉNÉRATION D'UN LIT CATALYTIQUE DÉSACTIVÉ DANS LE CADRE D'UNE DÉSHYDROGÉNATION PARTIELLE D'UN HYDROCARBURE À CATALYSE HÉTÉROGÈNE

(84) Benannte Vertragsstaaten:
**AT BE BG CH CY CZ DE DK EE ES FI FR GB GR HR HU IE IS IT LI LT LU LV MC MT NL NO PL PT RO SE SI SK TR**

(30) Priorität: **06.02.2007 DE 102007006647**
**06.02.2007 US 888366 P**

(43) Veröffentlichungstag der Anmeldung:
**18.11.2009 Patentblatt 2009/47**

(73) Patentinhaber: **BASF SE**
**67056 Ludwigshafen (DE)**

(72) Erfinder:
• **DIETERLE, Martin**
  **Jersey City, NJ 07302 (US)**
• **SCHINDLER, Götz-Peter**
  **67071 Ludwigshafen (DE)**
• **HORSTMANN, Catharina**
  **67056 Ludwigshafen (DE)**
• **MÜLLER-ENGEL, Klaus Joachim**
  **76297 Stutensee (DE)**

(56) Entgegenhaltungen:
**EP-A- 1 642 879    DE-A1- 10 028 582**

**Beschreibung**

Vorliegende Erfindung betrifft ein Verfahren zur Regenerierung eines im Rahmen einer heterogen katalysierten partiellen Dehydrierung eines zu dehydrierenden Kohlenwasserstoffs zu einem dehydrierten Kohlenwasserstoff deaktivierten Katalysatorbetts, das über einen Zeitraum t das Durchleiten eines molekularen Sauerstoff und Inertgas, aber keinen Kohlenwasserstoff enthaltenden Regeneriergases mit erhöhter Temperatur durch das deaktivierte Katalysatorbett mit der Maßgabe umfasst, dass der Gesamtgehalt $G^A$ des durch das Katalysatorbett geleiteten Regeneriergases an Kohlenoxiden (CO, $CO_2$) bei dessen Austritt aus dem Katalysatorbett im Verlauf der Regenerierung über den Zeitraum t wenigstens zeitweise größer ist, als der entsprechende Gehalt $G^E$, jeweils ausgedrückt in Vol.-% des Regeneriergasvolumens, desselben durch das Katalysatorbett geleiteten Regeneriergases bei dessen Eintritt in das Katalysatorbett und die Differenz $\Delta G = G^A - G^E$ bis zur Beendigung des Regenerierverfahrens einen Höchstwert $\Delta G^{max}$ durchläuft.

Der in dieser Anmeldung verwendete Begriff "dehydrierter Kohlenwasserstoff' soll Kohlenwasserstoffe umfassen, deren Moleküle wenigstens zwei ("zwei" sind anwendungstechnisch bevorzugt) Wasserstoffatome weniger enthalten, als die Moleküle eines zu dehydrierenden Kohlenwasserstoffs. Im übrigen soll der Begriff Kohlenwasserstoff Stoffe umfassen, deren Moleküle nur aus den Elementen Kohlenstoff und Wasserstoff aufgebaut sind.

Damit umfassen dehydrierte Kohlenwasserstoffe insbesondere acyclische und cyclische aliphatische Kohlenwasserstoffe mit einer oder mehreren C,C-Doppelbindungen im Molekül.

Beispiele für solche aliphatischen dehydrierten Kohlenwasserstoffe sind Propen, iso-Buten, Ethylen, 1-Buten, 2-Buten und Butadien sowie alle Pentene. D. h., zu den dehydrierten Kohlenwasserstoffen gehören insbesondere die einfach ungesättigten linearen (n-Alkene) oder verzweigten aliphatischen Kohlenwasserstoffe (z. B. iso-Alkene), sowie die Cycloalkene.

Ferner sollen die dehydrierten Kohlenwasserstoffe auch die Alkapolyene (z. B. Diene und Triene) umfassen, die mehr als eine Kohlenstoff-Kohlenstoff-Doppelbindung im Molekül enthalten. Dehydrierte Kohlenwasserstoffe sollen aber auch Kohlenwasserstoffverbindungen umfassen, die ausgehend von Alkylaromaten wie Ethylbenzol oder Isopropylbenzol durch Dehydrierung des Alkylsubstituenten erhältlich sind. Das sind z. B. Verbindungen wie Styrol oder $\alpha$-Methylstyrol.

Ganz generell bilden dehydrierte Kohlenwasserstoffe wertvolle Ausgangsverbindungen zur Synthese von z. B. funktionalisierten, radikalisch polymerisierbaren Verbindungen (z. B. Acrylsäure aus Propen oder Methacrylsäure aus iso-Buten und deren Polymerisationsprodukte). Beispielsweise können solche funktionalisierten Verbindungen durch Partialoxidation von dehydrierten Kohlenwasserstoffen erzeugt werden. Dehydrierte Kohlenwasserstoffe eignen sich aber auch zur Herstellung von Verbindungen wie Methyl-tert.-butyl-ether (Folgeprodukt von iso-Buten, das z. B. als Kraftstoffadditiv zur Einstellung der Oktanzahl geeignet ist). Dehydrierte Kohlenwasserstoffe können als solche aber auch selbst zur Polymerisation verwendet werden.

Als zu dehydrierende Kohlenwasserstoffe kommen in dieser Schrift insbesondere die acyclischen und cyclischen Alkane, aber auch Olefine (deren C,C-Doppelbindungszahl erhöht werden soll) in Betracht (als Beispiel sei die heterogen katalysierte partielle Dehydrierung von n-Butenen zu Butadien erwähnt).

D. h., der Begriff "zu dehydrierende Kohlenwasserstoffe" umfasst in dieser Patentanmeldung z. B. Kohlenwasserstoffe der Stöchiometrie $C_nH_{2n+2}$ mit n > 1 bis n ≤ 20, sowie der Stöchiometrie $C_nH_{2n}$ mit n > 1 bis n ≤ 20, sowie der Stöchiometrie $C_nH_{2n-2}$ mit n > 2 bis n ≤ 20, und n = ganzzahlig, insbesondere $C_2$- bis $C_{16}$-Alkane wie z. B. Ethan (zu Ethylen), Propan (zu Propylen), n-Butan, iso-Butan (zu iso-Buten), n-Pentan, isoPentan, n-Hexan, n-Heptan, n-Octan, n-Nonan, n-Decan, n-Undecan, n-Dodecan, n-Tridecan, n-Tetradecan, n-Pentadecan und n-Hexadecan.

Insbesondere aber gelten alle in dieser Schrift getroffenen Aussagen für $C_2$- bis $C_6$-Alkane als zu dehydrierende Kohlenwasserstoffe und ganz besonders für $C_2$- bis $C_4$-Alkane als zu dehydrierende Kohlenwasserstoffe. D. h., zu dehydrierende Kohlenwasserstoffe sind in dieser Schrift vor allem Ethan, Propan, n-Butan und iso-Butan, aber auch 1-Buten und 2-Buten sowie alle Pentane.

Vor allem gelten alle in dieser Schrift getroffenen Aussagen für Propan als zu dehydrierenden Kohlenwasserstoff und Propylen als resultierenden dehydrierten Kohlenwasserstoff, sowie für diese heterogen katalysierte Dehydrierung zu verwendende Katalysatoren.

Verfahren zur Herstellung von dehydrierten Kohlenwasserstoffen sind allgemein bekannt (vgl. z. B. WO 03/076370, DE-A 10 2004 032 129, EP-A 731 077, WO 01/96271, WO 01/96270, DE-A 103 16 039, WO 03/011804, WO 00/10961, EP-A 799 169, DE-A 102 45 585 sowie die deutschen Anmeldungen Nr. 10 2005 061 626, 10 2006 017 623 und 10 2006 035 718 und das Deutsche Aktenzeichen 102006024901.1).

Prinzipiell werden die Verfahren zur Herstellung von dehydrierten Kohlenwasserstoffen durch heterogen katalysierte partielle Dehydrierung wenigstens eines zu dehydrierenden Kohlenwasserstoffs in zwei Gruppen unterschieden: oxidative und nicht oxidative heterogen katalysierte partielle Dehydrierungen. Im Unterschied zur oxidativen heterogen katalysierten partiellen Dehydrierung erfolgt die nicht oxidative (in dieser Schrift auch als "konventionelle" heterogen katalysierte partielle Dehydrierung bezeichnet) heterogen katalysierte partielle Dehydrierung ohne Mitwirkung von Sauerstoff. D.h., der dem zu dehydrierenden Kohlenwasserstoff zu entreißende Wasserstoff wird unmittelbar als molekularer Wasserstoff entrissen und auch nicht in einem Folgeschritt mit Sauerstoff wenigstens teilweise zu Wasser oxidiert. Die Wärmetönung einer nicht oxidativen Dehydrierung ist somit in jedem Fall endotherm. Bei der oxidativen heterogen katalysierten partiellen Dehydrierung wird der dem zu dehydrierenden Kohlenwasserstoff zu entreißende molekulare Wasserstoff hingegen unter Mitwirkung von Sauerstoff entrissen. Dieses Entreißen kann unmittelbar als Wasser ($H_2O$) erfolgen (in diesem Fall spricht man verkürzt auch von einer heterogen katalysierten Oxidehydrierung; ihre Wärmetönung ist in jedem Fall exotherm). Das Entreißen kann aber auch zunächst als molekularer Wasserstoff erfolgen (d. h., nicht oxidativ bzw. konventionell), der dann in einem Folgeschritt teilweise oder vollständig mit Sauerstoff zu Wasser ($H_2O$) oxidiert wird (je nach Umfang der Wasserstofffolgeverbrennung kann die Bruttowärmetönung endotherm, exotherm oder neutral sein).

Allen vorgenannten heterogen katalysierten partiellen Dehydrierungen von zu dehydrierenden Kohlenwasserstoffen ist gemein, dass sie bei vergleichsweise hohen Reaktionstemperaturen durchgeführt werden müssen. Typische Reaktionstemperaturen können $\geq 250°C$, häufig $\geq 300°C$, oft $\geq 350°C$, oder $\geq 400°C$, bzw. $\geq 450°C$, oder $\geq 500°C$ betragen.

Weiterhin ist allen vorgenannten heterogen katalysierten partiellen Dehydrierungen von zu dehydrierenden Kohlenwasserstoffen gemein, dass im Verlauf ihres Langzeitbetriebes mit zunehmender Betriebszeit zunehmend noch höhere Reaktionstemperaturen erforderlich sind, um (unter ansonsten unveränderten Dehydrierbedingungen) den Dehydrierumsatz bei einmaligem Durchgang des Reaktionsgasgemischs durch den Reaktionsraum aufrechtzuerhalten. Dies rührt üblicherweise unter anderem daher, dass es auf der äußeren und inneren Oberfläche der verwendeten Katalysatoren zur Abscheidung von Kohlenstoffen und/oder hochsiedenden Kohlenwasserstoffen kommt, die den Zugang zu den aktiven Zentren der Katalysatoren blockieren, was umsatztechnisch normalerweise dadurch ausgeglichen wird, dass man die Wirksamkeit der jeweils verbliebenen zugänglichen Aktivzentren der Katalysatoren durch Temperaturerhöhung steigert.

Aus der WO 01/96008 ist bekannt, die heterogen katalysierte Dehydrierung von Zeit zu Zeit zu unterbrechen, um vorgenannte Abscheidungen dadurch zu entfernen, dass ein molekularen Sauerstoff und Inertgas, aber keinen Kohlenwasserstoff enthaltendes Regeneriergas bei erhöhter Temperatur durch das Katalysatorbett geleitet wird, welches die Abscheidungen zu Kohlenoxiden oxidiert. Nachteilig an der Lehre der WO 01/96008 ist, dass das Regeneriergas gemäß der Lehre der WO 01/96008 während der gesamten Regenerierdauer 5 % an molekularem Sauerstoff enthält. Das auf diese Weise innerhalb einer vorgegebenen Regenerierdauer zu erzielende Regenerierergebnis vermag nicht zu befriedigen.

Dies gilt in gleicher Weise für die in der DE-A 10 2006 029 790 für deaktivierte Oxidehydrierkatalysatoren empfohlene Deyhdrierweise, die sich an jene für Partialoxidationskatalysatoren anlehnt, wie sie in den Schriften DE-A 10351269, DE-A 10350812 und DE-A 10350822 beschrieben ist.

Aus der DE-A 10028582, der DE-A 10 2006 035 718, und der DE-A 10 2005 013 039 ist bekannt, die Regenerierung so durchzuführen, dass man bei einer Temperatur von 300 bis 600°C zunächst in ersten Regenerierungsstufen mit Stickstoff verdünnte Luft über das Katalysatorbett leitet. Die Katalysatorbelastung mit Regeneriergas kann dabei z.B. 50 bis 10000 $h^{-1}$ und der Sauerstoffgehalt des Regeneriergases 0,1 oder 0,5 bis 20 Vol.-% betragen. In nachfolgenden weiteren Regenerierstufen kann unter ansonsten gleichen Regenerierbedingungen als Regeneriergas Luft verwendet werden.

Nachteilig an beiden Schriften ist, dass sie keine konkrete Ausführungsform der empfohlenen Regenerierweise enthalten.

Die Aufgabe der vorliegenden Erfindung bestand daher darin, ein wie eingangs beschriebenes Regenerierverfahren zur Verfügung zu stellen, das einerseits eine erhöhte Effizienz und andererseits eine erhöhte Wirksamkeit bedingt.

Außerdem soll sich das Deaktivierungsverhalten des regenerierten Katalysatorbetts vom Deaktivierungsverhalten des frisch beschickten Katalysatorbetts im wesentlichen nicht unterscheiden. Dies ist bei den Verfahren gemäß Stand der Technik in der Regel nicht gegeben.

Demgemäß wurde ein Verfahren zur Regenerierung eines im Rahmen einer heterogen katalysierten partiellen Dehydrierung eines zu dehydrierenden Kohlenwasserstoffs zu einem dehydrierten Kohlenwasserstoff deaktivierten Katalysatorbetts, das über einen Zeitraum t das Durchleiten eines molekularen Sauerstoff und Inertgas, aber keinen Kohlenwasserstoff enthaltenden Regeneriergases mit erhöhter Temperatur durch das deaktivierte Katalysatorbett mit der Maßgabe umfasst, dass der Gesamtgehalt $G^A$ des durch das Katalysatorbett geleiteten Regeneriergases an Kohlenoxiden bei dessen Austritt aus dem Katalysatorbett im Verlauf der Regenerierung über den Zeitraum t wenigstens zeitweise größer ist, als der entsprechende Gehalt $G^E$, jeweils ausgedrückt in Vol.-% des Regeneriergasvolumens, desselben durch das Katalysatorbett geleiteten Regeneriergases bei dessen Eintritt in das Katalysatorbett und die Differenz $\Delta G = G^A - G^E$ bis zur Beendigung des Regenerierverfahrens einen Höchstwert $\Delta G^{max}$ durchläuft, gefunden, das dadurch gekennzeichnet ist, dass

a) $0,2 \text{ Vol.-\%} \leq \Delta G^{max} \leq 5 \text{ Vol.-\%}$ und

b) der Gehalt des durch das Katalysatorbett zu leitenden Regeneriergases an molekularem Sauerstoff, ausgedrückt in Vol.-% des Regeneriergasvolumens, innerhalb des Zeitraums t bis zur Beendigung des Regenerierverfahrens wenigstens dreimal erhöht wird und die jeweilige Erhöhung wenigstens 2 Vol.-% beträgt.

Erfindungsgemäß bevorzugt sind erfindungsgemäße Regenerierverfahren für die gilt 0,2 Vol.-% $\leq \Delta G^{max} \leq$ 4 Vol.-%, vorzugsweise 0,2 Vol.-% $\leq \Delta G^{max} \leq$ 3 Vol.-%, besonders bevorzugt 0,2 Vol.-% $\leq \Delta G^{max} \leq$ 2 Vol.-% und ganz besonders bevorzugt 0,3 Vol.-% $\leq \Delta G^{max} \leq$ 2 Vol.-%, bzw. 0,4 Vol.-% $\leq \Delta G^{max} \leq$ 2 Vol.-%. Günstig ist auch eine erfindungsgemäße Verfahrensweise mit 0,5 Vol.-% $\leq \Delta G^{max} \leq$ 2 Vol.-%, bzw. 0,4 Vol.-% $\leq \Delta G^{max} \leq$ 1,5 Vol.-% oder 0,5 Vol.-% $\leq \Delta G^{max} \leq$ 1,5 Vol.-%.

$\Delta G^{max}$ ist die höchste Zunahme des Gehalts des Regeneriergases an Kohlenoxiden, die das Regeneriergas beim regenerierenden Durchströmen des Regeneriergases durch das Katalysatorbett im Verlauf des erfindungsgemäßen Verfahrens (d.h., innerhalb des Zeitraums t) erfährt.

Der Zeitraum t des erfindungsgemäßen Verfahrens kann sich über wenige Minuten bis Stunden (z.B. $\geq$ 1 h, oder $\geq$ 2 h, oder $\geq$ 3 h) oder auch über einige Tage (z.B. $\geq$ 24 h, oder $\geq$ 48 h, oder $\geq$ 72 h) erstrecken. In der Regel wird das erfindungsgemäße Verfahren nicht mehr als 14 Tage beansprucht. Selbstverständlich kann das erfindungsgemäße Verfahren auch temporär unterbrochen und anschließend wieder aufgenommen werden.

In der Regel wird man das erfindungsgemäße Verfahren wenigstens so lange betreiben, bis der Gehalt des deaktivierten Dehydrierkatalysatorbetts an darin enthaltenen abgeschiedenen Kohlenstoff enthaltenden Bestandteile nur noch $\leq$ 75 Gew.-% (vorzugsweise $\leq$ 50 Gew.-%, besonders bevorzugt $\leq$ 25 Gew.-%, besser $\leq$ 10 Gew.-%, noch besser $\leq$ 5 Gew.-%, bzw. $\leq$ 1 Gew.-% und ganz besonders bevorzugt $\leq$0,1 Gew.-%, oder 0 Gew.-%) der zum Verfahrensbeginn darin enthaltenen abgeschiedenen Kohlenstoff enthaltenden Bestandteile (jeweils gerechnet als in diesen Bestandteilen enthaltene Gewichtsmenge an Kohlenstoff) beträgt.

Die erfindungsgemäß erforderliche wenigstens dreimalige Erhöhung des Regeneriergasgehaltes an molekularem Sauerstoff innerhalb des Zeitraums t kann z.B. als eine jeweils einmalige sprunghafte Erhöhung erfolgen. Sie kann aber auch als eine Aufeinanderfolge kleinerer sprunghafter Erhöhungen und/oder auch in Gestalt einer kontinuierlichen Erhöhung verwirklicht werden. Ist im Rahmen einer kontinuierlichen Erhöhung des Regeneriergasgehaltes an molekularem Sauerstoff eine Erhöhung von 2 Vol.-% erreicht, so ist damit das Erfordernis einer einmaligen erfindungsgemäßen Erhöhung des Regeneriergasgehaltes an molekularem Sauerstoff erfüllt.

Bis zur Beendigung des erfindungsgemäßen Verfahrens zur Regenerierung (d.h., bis zum Zeitpunkt t) muss der Sauerstoffgehalt des Regeneriergases erfindungsgemäß somit insgesamt um wenigstens 6 Vol.-% erhöht werden. Erfindungsgemäß bevorzugt beträgt vorgenannte (integrale) Erhöhung wenigsten 8 Vol.-%, besonders bevorzugt wenigstens 10 Vol.-%, ganz besonders bevorzugt wenigstens 12 Vol.-% und noch besser wenigstens 14 Vol.-% oder wenigstens 16 Vol.-%. Erfindungsgemäß günstig ist auch eine vorgenannte (integrale) Insgesamterhöhung des Sauerstoffgehaltes des Regeneriergases von wenigstens 18 Vol.-%, oder wenigstens 19 Vol.-%, oder wenigstens 20 Vol.-%. In der Regel beträgt die Insgesamterhöhung aber nicht mehr als 21 Vol.-%.

D. h., vorzugsweise wird der Sauerstoffgehalt des Regeneriergases beim erfindungsgemäßen Verfahren innerhalb des Zeitraums t wenigstens dreimal oder viermal, besser wenigstens fünfmal oder wenigstens siebenmal, noch besser wenigstens achtmal oder wenigstens neunmal um wenigstens 2 Vol.-% erhöht.

In der Regel wird man eine Erhöhung dann vornehmen, wenn unter ansonsten gleichbleibenden Regenerierbedingungen $\Delta G$ gegen Null geht (z.B. den Wert 0,01 Vol.-% unterschreitet).

Weiterhin ist es für das erfindungsgemäße Verfahren günstig, wenn der Gehalt des durch das Katalysatorbett zu führenden Regeneriergases an molekularem Sauerstoff bei Beginn (am Anfang) des erfindungsgemäßen Verfahrens $\leq$15 Vol.-%, vorzugsweise $\leq$10 Vol.-%, besonders bevorzugt $\leq$ 5 Vol.-%, ganz besonders bevorzugt $\leq$ 3 Vol.-% und noch besser $\leq$2 Vol.-% bzw. $\leq$1 Vol.-% beträgt. Normalerweise wird der Gehalt des durch das Katalysatorbett zu führenden Regeneriergases an molekularem Sauerstoff bei Beginn des erfindungsgemäßen Verfahrens jedoch $\geq$0,25 Vol.-%, meist $\geq$ 0,5 Vol.-% und vielfach $\geq$ 0,75 Vol.-% betragen.

Häufig wird sich der Gehalt des durch das Katalysatorbett zu führenden Regeneriergases an molekularem Sauerstoff im Verlauf des erfindungsgemäßen Verfahrens somit im Bereich von 0,25 Vol.-% bis 21 Vol.-% bewegen.

Die Temperatur des molekularen Sauerstoff enthaltenden Regeneriergases beträgt beim Eintritt desselben in das deaktivierte Katalysatorbett bei der Durchführung des erfindungsgemäßen Verfahrens anwendungstechnisch zweckmäßig 200 bis 700°C, bevorzugt 300 bis 600°C, besonders bevorzugt 400 bis 600°C, ganz besonders bevorzugt 420 bis 550°C bzw. 440 bis 470°C.

Erfindungsgemäß vorteilhaft sollte das erfindungsgemäße Verfahren so durchgeführt werden, dass die Temperatur des Regeneriergases beim Austritt aus dem Katalysatorbett noch unterhalb derjenigen Temperatur liegt, die im Verlauf der heterogen katalysierten partiellen Dehydrierung, die die Deaktivierung des Katalysatorbetts bedingt hat, als höchste Temperatur im Katalysatorbett aufgetreten ist (bzw. wenigstens unterhalb des 1,5-fachen Wertes dieser Temperatur). In vorteilhafter Weise liegt die im Rahmen des erfindungsgemäßen Regenerierverfahrens im Katalysatorbett höchste auftretende Temperatur bei Werten $\leq$ 700°C, vorzugsweise $\leq$ 650°C, besonders bevorzugt $\leq$600°C und ganz besonders bevorzugt $\leq$ 575°C bzw. $\leq$ 550°C.

Als erfindungsgemäß zweckmäßig hat es sich erweisen, wenn die im Rahmen des erfindungsgemäßen Regenerierverfahrens (im Zeitraum t) im Katalysatorbett auftretenden höchsten Temperaturen T, bezogen auf die im Rahmen der die Deaktivierung des Katalysatorbetts bedingenden heterogen katalysierten partiellen Dehydrierung im Katalysatorbett aufgetretene höchste Temperatur $T^{max}$ folgende Bedingungen erfüllen: $0,5 \cdot T^{max} \leq T \leq 1,5 \cdot T^{max}$, bzw., $0,5 \cdot T^{max} \leq T \leq 1 \cdot T^{max}$.

Mit Vorteil wird man beim erfindungsgemäßen Regenerierverfahren so vorgehen, dass man die Temperatur des molekularen Sauerstoff enthaltenden Regeneriergases beim Eintritt in das zu regenerierende Katalysatorbett im Verlauf des erfindungsgemäßen Dehydrierverfahrens (d.h., innerhalb des Zeitraums t) wenigstens einmal erhöht.

Unter der Belastung des Katalysatorbetts mit Regeneriergas oder mit Reaktionsgas soll in dieser Schrift die Menge an Regeneriergas oder Reaktionsgas in Normlitern (= NI; das Volumen in Litern, das die entsprechende Regeneriergas- oder Reaktionsgasmenge bei Normalbedingungen, d.h., bei 0°C und 1 atm einnehmen würde) verstanden, die pro Stunde durch einen Liter Katalysatorbett geführt wird. Reine Inertmaterialschüttungen werden dabei in beiden Fällen nicht als zum Katalysatorbett gehörig miteingerechnet. Besteht das Katalysatorbett aus mit reinem Inertmaterial verdünntem Katalysator, so wird mit Bezug auf eine Berechnung der Belastung des Katalysatorbetts mit Regeneriergas dieses Inertmaterial als zum Katalysatorbett zugehörig gerechnet, mit Bezug auf eine Berechnung der Belastung des Katalysatorbetts mit Reaktionsgas jedoch nicht. Für sonstige Bedürfnisse des erfindungsgemäßen Verfahrens werden sowohl reine Inertmaterialschüttungen als auch inerte Verdünnungen zum Katalysatorbett zugehörig betrachtet.

Die Belastung kann auch nur auf einen Bestandteil des Regeneriergases oder nur auf einen Bestandteil des Reaktionsgases bezogen sein. Dann ist es die Menge dieses Bestandteils in NI/(I·h) (oder kurz: NI/I·h, oder noch kürzer: $h^{-1}$), die pro Stunde durch einen Liter des Katalysatorbetts geführt wird.

Erfindungsgemäß vorteilhaft werden beim erfindungsgemäßen Regenerierverfahren Belastungen des Katalysatorbetts mit Regeneriergas angewendet, die $\geq 500$ NI/I·h betragen. Hohe Belastungen des Katalysatorbetts mit Regeneriergas bedingen in der Regel einen erhöhten Anteil an Inertgas im Regeneriergas. Ein solcher ist erfindungsgemäß insofern vorteilhaft, als er erhöhten Kohlenoxidkonzentrationen (insbesondere erhöhten Kohlenmonoxidkonzentrationen) im Regeneriergas entgegenwirkt. Erhöhte Kohlenmonoxidgehalte im Regeneriergas erweisen sich erfindungsgemäß insofern als nachteilig, als sie über eine ausgeprägt chemisch reduzierende Wirkung verfügen, die sich auf die Reaktivierung des Katalysatorbetts negativ auswirkt. Dies trifft auf das Kohlendioxid zwar nur in wesentlich schwächer ausgeprägtem Umfang zu, allerdings vermag dieses mit noch vorhandenen Kohlenstoffablagerungen zu CO zu reagieren und auf diese Weise insbesondere lokal erhöhte Kohlenmonoxidkonzentrationen zu bilden. Darüber hinaus ist die Wärmekapazität des Inertgasanteils förderlich für den Austrag der im Rahmen des Regenerierverfahrens gebildeten Reaktionswärme.

Im Regelfall wird die Belastung des Katalysatorbetts mit Regeneriergas jedoch $\leq 50000$ NI/I·h betragen, da mit einer zunehmenden Belastung des Katalysatorbetts mit Regeneriergas ein erhöhter Druckverlust einhergeht.

Beim erfindungsgemäßen Verfahren bevorzugte Belastungen B des Katalysatorbetts mit Regeneriergas betragen daher 1000 NI/I·h $\leq B \leq$ 40000 NI/I·h, besonders bevorzugt 2000 NI/I·h $\leq B \leq$ 30000 NI/I·h, besonders bevorzugt 3000 NI/I·h $\leq B \leq$ 20000 NI/I·h und ganz besonders bevorzugt 4000 NI/I·h $\leq B \leq$ 15000 NI/I·h, bzw. 5000 NI/I·h $\leq B \leq$ 10000 NI/I·h. Bei vergleichsweise geringen Gehalten des deaktivierten Katalysatorbetts an Kohlenstoff enthaltenden Abscheidungen wird die Belastung B erfindungsgemäß vorteilhaft niedrig gewählt (z. B. 300 NI/I·h bis 700 NI/I·h).

Erfindungsgemäß vorteilhaft durchläuft die Belastung B des Katalysatorbetts mit Regeneriergas im Verlauf des erfindungsgemäßen Regenerierverfahrens (innerhalb des Zeitraums t) ein Maximum.

D.h. z.B., man beginnt das erfindungsgemäße Verfahren zunächst mit gemäßigter Belastung, um diese im Verlauf des Regenerierverfahrens allmählich bis zu einem Maximalwert zu erhöhen. Von diesem ausgehend wird die Belastung des Katalysatorbetts mit Regeneriergas nachfolgend wieder gesenkt.

Typische Anfangsbelastungen können z.B. 4000 bis 8000 NI/I·h betragen. Typische Maximalbelastungen können z.B. 7000 bis 11000 NI/I·h betragen. Typische Endbelastungen können z.B. 500 bis 4000 NI/I·h betragen. Bei vergleichsweise geringen Gehalten des deaktivierten Katalysatorbetts an Kohlenstoff enthaltenden Abscheidungen können vorgenannte Werte auch z. B. 400 bis 600 NI/I·h (Anfang), 1000 bis 3000 NI/I·h (Maximum) und 500 bis 2000 NI/I·h (Ende) betragen.

Wird beim erfindungsgemäßen Verfahren Luft als Sauerstoffquelle für den im Regeneriergas enthaltenen molekularen Sauerstoff verwendet, lässt sich das vorbeschriebene Belastungsprofil des zu regenerierenden Katalysatorbetts in einfacher Weise z.B. wie folgt verwirklichen. Man beginnt die Regenerierung mit einem Gasgemischstrom, der durch Zusammenführen von einem Luftstrom und einem überwiegend (zu mehr als 50 Vol.-%) aus Inertgas bestehenden Gasstrom erzeugt wird. Anschließend behält man letzteren im wesentlichen bei und erhöht über die Regenerierdauer allmählich den Luftstrom (die pro Zeiteinheit zugeführte Luftmenge). Dies bewirkt mit zunehmender Regenerierdauer gleichzeitig eine Erhöhung des Sauerstoffgehaltes im Regeneriergas. Hat der Luftstrom seinen Maximalwert erreicht, beginnt man nachfolgend den überwiegend aus Inertgas bestehenden Gasstrom zu vermindern. Dies bedingt einerseits eine weitere Erhöhung des Sauerstoffgehaltes im Regeneriergas und verringert andererseits die Belastung des Katalysatorbetts mit Regeneriergas. Erfindungsgemäß vorteilhaft besteht das Regeneriergas zum Ende des erfindungsgemäßen Regenerierverfahrens dann nur noch aus dem Luftstrom. Dieser weist anwendungstechnisch zweckmäßig dann auch die im Verlauf des Regenerierverfahrens höchste angewandte Temperatur des Regeneriergases beim Eintritt desselben in das zu regenerierende Katalysatorbett auf.

Mit Vorteil wird dieser Regeneriergasstrom, der das höchste Oxidationspotential aufweist, anschließend noch einige Zeit beibehalten, um diese maximale oxidierende Wirkung zu entfalten. Dies ist ganz generell vorteilhaft beim erfindungsgemäßen Verfahren. D.h., in der Regel wird man beim erfindungsgemäßen Verfahren mit demjenigen Regeneriergasstrom, der das höchste Oxidationspotential, (d.h., üblicherweise den höchsten Gehalt an molekularem Sauerstoff sowie die höchste Temperatur beim Eintritt in das Katalysatorbett) aufweist, das erfindungsgemäße Verfahren abschließen (umgekehrt wird man das erfindungsgemäße Verfahren bevorzugt mit dem Regeneriergasstrom beginnen, der im Verlauf des erfindungsgemäßen Verfahrens das geringste Oxidationspotential aufweist).

Unter einem Inertgas (für die erfindungsgemäße Regenerierung bzw. heterogen katalysierte partielle Dehydrierung) versteht man in dieser Schrift von molekularem Sauerstoff und von Kohlenwasserstoffen verschiedene Gase, die als Bestandteil von Regeneriergas bzw. Reaktionsgas beim regenerierenden bzw. dehydrierenden Durchströmen des Katalysatorfestbetts zu wenigstens 95 mol-%, vorzugsweise zu wenigstens 97 mol-% und besonders bevorzugt zu wenigstens 99 mol-% (jeder Bestandteil des Inertgases für sich betrachtet) chemisch unverändert erhalten bleiben (unter den jeweiligen Regenerier- bzw. Dehydrierbedingungen). Beispiele für erfindungsgemäß geeignete Inertgase (inerte Verdünnungsgase) sind (sowohl für den Fall der Regenerierung als auch den Fall der heterogen katalysierten partiellen Dehydrierung) $N_2$, He, Ne, Ar sowie $H_2O$ und mit der bereits angesprochenen Einschränkung $CO_2$, sowie Mischungen aus zwei oder mehr dieser Gase. Erfindungsgemäß ganz besonders bevorzugtes Inertgas ist, insbesondere für den Fall der erfindungsgemäßen Regenerierung, molekularer Stickstoff.

Vorstehende Inertgasdefinition gilt so auch für die Reaktionsgase anderer, z. B. heterogen katalysierter, Gasphasenreaktionen.

Über weite Strecken des erfindungsgemäßen Regenerierverfahrens enthält das Regeneriergas erfindungsgemäß zweckmäßig mehr (bzw. ≥) als 78 Vol.-%, häufig sogar mehr (bzw. ≥) als 80 Vol.-% oder mehr (bzw. ≥) als 90 Vol.-% an Inertgas (in vielen Fällen $N_2$).

Gleichzeitig soll das Regeneriergas beim erfindungsgemäßen Verfahren dem zu regenerierenden Katalysatorfestbett mit erhöhter Temperatur zugeführt werden. Um sowohl den diesbezüglich erforderlichen Bedarf an Inertgas als auch den diesbezüglich erforderlichen Bedarf an Energie zu minimieren, wird man beim erfindungsgemäßen Verfahren das Regeneriergas erfindungsgemäß vorteilhaft wenigstens teilweise im Kreis (führen) fahren und als Bestandteil von frisch durch das Katalysatorbett zu führendem Regeneriergas wiederverwenden.

D. h., das aus dem zu regenerierenden Katalysatorbett austretende Regeneriergas wird (wenigstens teilweise) häufig größtenteils (grundsätzlich kann der rückgeführte Regeneriergasanteil, bezogen auf den aus dem Katalysatorbett austretenden Regeneriergasvolumenstrom, z. B. 10 bis 97 Vol.-% (bzw. 10 bis 95 Vol.-% oder 10 bis 90 Vol.-%) oder 30 bis 60 Vol.-% betragen) an den Reaktoreingang rückgeführt und mit soviel von dem als Sauerstoffquelle verwendeten Gas (z.B. Luft oder reiner molekularer Sauerstoff oder ein Gemisch aus beiden) ergänzt, dass das resultierende Gemisch den für das Regeneriergas erwünschten Gehalt an molekularem Sauerstoff aufweist. Eine dieser Ergänzungsmenge im wesentlichen entsprechende Volumenmenge an aus dem Katalysatorbett austretendem Regeneriergas kann vorab der Rückführung des Regeneriergases abgetrennt und seiner Entsorgung zugeführt werden (z.B. Verbrennung in einer Fackel oder in einem Brenner zu Zwecken der Energiegewinnung). Strebt man eine Verringerung der Belastung des zu regenerierenden Katalysatorbetts mit Regeneriergas an, wird man den vorab seiner Rückführung abgetrennten Anteil an Regeneriergas in entsprechender Weise zunehmend größer wählen.

Zum Zweck der Rückführung des Regeneriergases an den Katalysatorbetteingang ist normalerweise ein Gebläse (vorzugsweise ein Radialgebläse) ausreichend, um den beim Durchgang durch das Katalysatorbett erlittenen Druckverlust wieder auszugleichen. Bei Katalysatorbetten, die einen besonders hohen Druckverlust bedingen, kann die Rückverdichtung auch mit Hilfe eines Radialverdichters vorgenommen werden. Die zur Erzeugung des neuen Regeneriergases zusätzlich erforderliche Sauerstoffquelle steht in der Regel aus anderen Quellen mit dem erforderlichen Druck zur Verfügung. Gegebenenfalls erfolgt ihre Verdichtung aber auch bereits im Gemisch mit rückzuführendem Regeneriergas.

Anwendungstechnisch zweckmäßig wird man das aus dem zu regenerierenden Katalysatorbett austretende Regeneriergas, das normalerweise eine erhöhte Temperatur aufweist, mit dem dem zu regenerierenden Katalysatorbett zuzuführenden Regeneriergas in indirektem Wärmeaustausch durch einen entsprechenden Wärmeaustauscher führen. Das erstere kühlt dabei ab und das letztere wird auf die gewünschte Eingangstemperatur erwärmt. Üblicherweise erfolgt der indirekte Wärmeaustausch vorab der Rückverdichtung. Für den Fall, dass im Rahmen der Abkühlung Wasser auskondensiert, wird selbiges anwendungstechnisch vorteilhaft mit Hilfe von Tropfenabscheidern abgeschieden und aus dem Regenerierkreis herausgeführt.

Zu einer solchen Wasserkondensation kann es auch dann kommen, wenn man das rückzuverdichtende Regeneriergas zur Vermeidung zu hoher Temperaturen beim Verdichten zusätzlich z.B. in einem Luftkühler oder in einem mit Oberflächenwasser gekühlten Wärmeaustauscher abkühlt. Auch solchermaßen auskondensiertes Wasser würde man erfindungsgemäß zweckmäßig vorab einer Rückverdichtung abtrennen.

Selbstredend kann der Gehalt des durch das Katalysatorbett zu führenden Regeneriergases an molekularem Sauerstoff im Verlauf des erfindungsgemäßen Regenerierverfahrens auch oberhalb des Gehaltes an molekularem Sauerstoff von Luft gewählt werden. Beispielsweise könnte zum Ende des erfindungsgemäßen Regenerierverfahrens auch reiner molekularer Sauerstoff als Regeneriergas verwendet werden. Aus Gründen der Wirtschaftlichkeit wird der Gehalt an molekularem Sauerstoff des durch das Katalysatorbett zu führenden Regeneriergases normalerweise denjenigen von Luft nicht übersteigen.

Erfindungsgemäß vorteilhaft wird der CO-Gehalt des durch das Katalysatorbett zu führenden Regeneriergases selbst bei vorstehend beschriebener Kreisgasfahrweise beim Eintritt des Regeneriergases in das Katalysatorbett $\leq 3$ Vol.-%, bevorzugt $\leq 2$ Vol.-%, besonders bevorzugt $\leq 1$ Vol.-%, und ganz besonders bevorzugt $\leq 0,1$ Vol.-% bzw. $\leq 0,05$ Vol.-% betragen. Dies nicht zuletzt deshalb, weil erhöhte CO-Konzentrationen im Regeneriergas den erfindungsgemäßen Zielsetzungen in besonderer Weise entgegenzuwirken scheinen.

Ganz generell beträgt der Anteil der von Inertgas, Kohlenoxiden und molekularem Sauerstoff verschiedenen Bestandteile am Regeneriergas bei dessen Eintritt in das Katalysatorbett erfindungsgemäß vorteilhaft $\leq 2$ Vol.-%, besser $\leq 1$ Vol.-%, bevorzugt $\leq 0,5$ Vol.-%, ganz besonders bevorzugt $\leq 0,1$ Vol.-% und am besten 0 Vol.-%.

Grundsätzlich kann das Regeneriergas beim erfindungsgemäßen Verfahren durch das zu regenerierende Katalysatorbett gesaugt werden. In diesem Fall liegt der Arbeitsdruck beim erfindungsgemäßen Regenerierverfahren unterhalb von Atmosphärendruck. Erfindungsgemäß bevorzugt sind beim erfindungsgemäßen Regenerierverfahren Arbeitsdrucke, die 1 bis 10 bar, vorzugsweise 1 bis 6 bar, und besonders bevorzugt 1,5 bis 4 bar oberhalb von Atmosphärendruck liegen. Dies liegt nicht zuletzt darin begründet, dass ein Arbeiten bei überatmosphärischen Arbeitsdrucken in vorteilhafter Weise erhöhte $O_2$-Partialdrucke ermöglicht.

Das erfindungsgemäße Verfahren eignet sich sowohl im Fall von deaktivierten Katalysatorbetten die in (im Reaktionsraum von) adiabaten, d.h., adiabat gestalteten (mit gegenüber ihrer äußeren Umgebung wärmeisolierten Reaktionsräumen) Reaktoren untergebracht sind, als auch im Fall von deaktivierten Katalysatorbetten, die in isothermen, d.h., isotherm gestalteten Reaktoren (ermöglichen eine Lenkung der Temperatur im Reaktionsraum durch Wärmeaustausch mit außerhalb des Reaktionsraumes geführten Wärmeträgern) untergebracht sind (das Katalysatorbett befindet sich jeweils im Reaktionsraum).

An dieser Stelle sei festgehalten, dass beim erfindungsgemäßen Regenerierverfahren beim Durchgang des Regeneriergases durch das zu regenerierende Katalysatorbett die Temperatur im Katalysatorbett vorzugsweise im wesentlichen nicht unter die Eintrittstemperatur des Regeneriergases ins Katalysatorbett fällt. Im Fall eines in einem adiabaten Reaktor befindlichen zu regenerierenden Katalysatorbetts ist diese Voraussetzung durch die Adiabasie des Reaktors normalerweise weitgehend erfüllt. Im Fall eines in einem isothermen Reaktor befindlichen Katalysatorbetts muss die Temperatur des externen Wärmeträgermediums entsprechend eingestellt werden.

Umgekehrt sollte, insbesondere bei in adiabaten Reaktoren befindlichen zu reaktivierenden Katalysatorbetten, der Unterschied zwischen der Temperatur des Regeneriergases beim Eintritt in das Katalysatorbett, $T^E$, und der Temperatur desselben Regeneriergases beim Austritt aus dem Katalysatorbett, $T^A$, d.h. $T^A$-$T^E$. beim erfindungsgemäßen Verfahren $\leq 250°C$, besser $\leq 150°C$, vorzugsweise $\leq 100°C$, betragen. Erfindungsgemäß günstig ist eine Differenz $T^A$-$T^E$ von etwa 50°C.

Grundsätzlich kann das Katalysatorbett beim erfindungsgemäßen Verfahren sowohl ein Katalysatorfestbett, als auch ein Katalysatorwirbelbett sein. Erfindungsgemäß bevorzugt handelt es sich beim erfindungsgemäß zu regenerierenden Katalysatorbett um ein Katalysatorfestbett (alle Aussagen dieser Patentanmeldung gelten daher insbesondere für den Fall eines Katalysatorfestbetts). Die erfindungsgemäße Verfahrensweise ist dabei dann von besonderer Relevanz, wenn das Schüttvolumen des Katalysatorfestbetts $\geq 50\ I$ und $\leq 10000\ m^3$, in der Regel $\leq 5000\ m^3$ und meist $\leq 1000\ m^3$ bzw. $\leq 500\ m^3$ oder $\leq 50\ m^3$ beträgt. Dies gilt insbesondere dann, wenn sich das Katalysatorfestbett in einem adiabat gestalteten Reaktor bzw. dessen Reaktionsraum befindet. Dies ist nicht zuletzt dadurch bedingt, dass mit zunehmendem Schüttvolumen des Katalysatorfestbetts die Wärmekapazität des Reaktormaterials zunehmend an Bedeutung und Einfluss verliert.

Die Notwendigkeit der Durchführung einer partiellen Dehydrierung eines zu dehydrierenden Kohlenwasserstoffs an im festen Zustand befindlichen selektiv wirkenden Dehydrierkatalysatoren ist dadurch bedingt, dass die Dehydrierung (Spaltung von C-H) mit der thermischen Spaltung bzw. Crackung (Spaltung von C-C) kinetisch konkurriert.

Infolge der selektiv wirkenden Katalysatoren entsteht im Fall einer heterogen katalysierten Dehydrierung eines zu dehydrierenden Kohlenwasserstoffs (z.B. Propan) der dehydrierte Kohlenwasserstoff (z.B. Propylen) mit erhöhter Selektivität. Nebenprodukte wie z.B. Methan, Ethylen und Ethan werden dann nur in vergleichsweise untergeordneten Mengen gebildet.

Die Anwendung des erfindungsgemäßen Regenerierverfahrens kann bei allen im Stand der Technik bekannten Dehydrierkatalysatoren erfolgen.

Für den Fall von von heterogen katalysierten Oxidehydrierungen verschiedenen heterogen katalysieren Deyhdrierungen lassen sich diese bekannten Dehydrierkatalysatoren grob in zwei Gruppen unterteilen. Nämlich in solche, die oxidischer Natur sind (z.B. Chromoxid und/oder Aluminiumoxid) und in solche, die aus wenigstens einem auf einem, in der Regel oxidischen (z.B. Zirkondioxid, Aluminiumoxid, Siliziumdioxid, Titandioxid, Magnesiumoxid, Lanthanoxid und/oder Ceroxid) Träger abgeschiedenen Metall (in einer elementaren Form) bestehen. In der Regel handelt es sich bei wenigstens einem der abgeschiedenen Metalle um ein vergleichsweise edles Metall (z.B. um ein Element aus der Gruppe umfassend Cu, Ag, Au, Zn, Cd, Hg sowie die Elemente der dritten Nebengruppe (z.B. Ru, Rh, Pd, Os, Ir, Pt), oder um ein Element aus der Platingruppe, z.B. um Platin und/oder Palladium). Zu vorgenannten Katalysatoren gehören somit z.B. solche, die auf dem (in der Regel oxidischen) Katalysatorträger ein oder mehrere Elemente, ausgewählt aus der Gruppe bestehend aus den Elementen der VIII und/oder VI. Nebengruppe, und gegebenenfalls ein oder mehrere weitere Elemente, ausgewählt aus der Gruppe bestehend aus den Elementen der I. und II. Hauptgruppe, der III. Nebengruppe einschließlich der Lanthaniden, der III. Hauptgruppe, Rhenium, Zink und Zinn enthalten.

Als dehydrieraktive Elemente sind insbesondere Metalle der VIII. Nebengruppe geeignet, vorzugsweise die Edelmetalle Platin und Palladium, besonders bevorzugt Platin. Wenn ein Edelmetall als dehydrieraktives Element eingesetzt wird, können zusätzliche Metalle, die das Sintern des Edelmetalls verlangsamen, wie Re und/oder Sn vorhanden sein. Als weitere Elemente kommen solche in Frage, von denen bekannt ist, dass sie die Acidität der Katalysatoroberfläche beeinflussen oder Edelmetalle gegen Sintern stabilisieren können. Solche weiteren Elemente sind Elemente der I. und II. Hauptgruppe, nämlich Li, Na, K, Rb, Cs, Mg, Ca, Sr und Ba sowie Elemente der III. Nebengruppe wie insbesondere Y und La, einschließlich der Lanthaniden. Als wirksam hat sich auch Zn erwiesen. Anstelle eines Edelmetalls (oder als Ergänzung zu diesem) können auch dehydrieraktive Metalle der VI. Nebengruppe, insbesondere Chrom oder Molybdän, auf dem Katalysatorträger vorliegen. Für die Notwendigkeiten dieser Schrift sollen die beiden vorgenannten Gruppen als "oxidische Dehydrierkatalysatoren" und als "metallische Dehydrierkatalysatoren" gegeneinander differenziert werden. Das erfindungsgemäße Regenerierverfahren ist somit insbesondere auf alle Dehydrierkatalysatoren anwendbar, die in der DE-A 10 2006 035 718, der WO 01/96270, der DE-A 10219879, der EP-A 731 077, der DE-A 10131297, der WO 99/46039, der US-A 4,788,371, der EP-A 705 136, der WO 99/29420, der US-A 4,220,091, der US-A 5,430,220, der US-A 5,877,369, der EP-A 117 146, der DE-A 19937196, der DE-A 19937105, der US-A 3,670,044, der US-A 6,566,573, der WO 01/83405 und der WO 94/29021 sowie dem in diesen Schriften zitierten Stand der Technik offenbart werden. Dies trifft insbesondere dann zu, wenn diese Dehydrierkatalysatoren im Verlauf der in diesen Schriften beschriebenen heterogen katalysierten Dehydrierungen von zu dehydrierenden Kohlenwasserstoffen deaktiviert worden sind.

Im Unterschied zum Vorgenannten kommen als Katalysatoren für heterogen katalysierte Oxidehydrierungen im wesentlichen ausschließlich Katalysatoren in Betracht, die oxidischer Natur sind (z.B. solche auf der Grundlage von MoVNb-Oxiden, oder auf der Grundlage von Vanadylpyrophosphat (zusätzlich können sie Promotoren enthalten).

Das erfindungsgemäße Regenerierverfahren ist somit zusätzlich für alle Oxidehydrierkatalysatoren geeignet, die in den Schriften US-A 4,788,371, CN-A 1073893, Catalysis Letters 23 (1994), 103-106, W. Zhang, Gaodeng Xuexiao Huaxue Xuebao, 14 (1993) 566, Z. Huang, Shiyon Huagong, 21 (1992) 592, WO 97/36849, DE-A 19753817, US-A 3,862,256, US-A 3,887,631, DE-A 19530454, US-A 4,341,664, J. of Catalysis 167, 560-569 (1997), J. of Catalysis 167, 550-559 (1997), Topics in Catalysis 3 (1996) 265-275, US-A 5,086,032, Catalysis Letters 10 (1991), 181-192, Ind. Eng. Chem. Res. 1996, 35, 14-18, US-A 4,255,284, Applied Catalysis A: General, 100 (1993), 111-130, J. of Catalysis 148, 56-67 (1994), V. Cortés Corberán und S. Vic Bellón (Ed.), New Developments in Selective Oxidation II, 1994, Elsevier Science B.V., S. 305-313, 3rd World Congress on Oxidation Catalysis, R.K. Grasselli, S.T. Oyama, A.M. Gaffney and J.E. Lyons (Ed.), 1997, Elsevier Science B.V., S. 375ff oder in DE-A 19837520, DE-A 19837517, DE-A 19837519, DE-A 19837518, EP-A 938 463, EP-A 167 109,

DE-A 19838312 und DE-A 19753817 offenbart werden. Dies trifft insbesondere dann zu, wenn diese Oxidehydrierkatalysatoren im Verlauf der in diesen Schriften beschriebenen heterogen katalysierten Oxidehydrierungen von zu dehydrierenden Kohlenwasserstoffen deaktiviert worden sind.

Unter Einbezug der oxidischen Oxidehydrierkatalysatoren lässt sich die Gesamtmannigfaltigkeit von bekannten Dehydrierkatalysatoren somit auch insgesamt in "oxidische Dehydrierkatalysatoren" und in "metallische Dehydrierkatalysatoren" aufteilen.

Bei den oxidischen Dehydrierkatalysatoren wird man die erfindungsgemäße Regenerierung in der Regel dann beenden, wenn ΔG beim Durchleiten des Regeneriergases mit dem höchsten Oxidationspotential (d.h., mit der höchsten ins Auge gefassten Eintrittstemperatur des Regeneriergas in das Katalysatorbett und mit dem höchstens ins Auge gefassten Gehalt des Regeneriergases an molekularem Sauerstoff) durch das zu regenerierende Katalysatorbett im wesentlichen gegen Null geht. Um bezüglich dieses Zeitpunktes über erhöhte Sicherheit zu verfügen, ist es anwendungstechnisch zweckmäßig, beim Durchleiten des Regeneriergases mit dem höchsten Oxidationspotential durch das Katalysatorbett von Zeit zu Zeit den Arbeitsdruck zu senken (z.B. von 3,5 bar überatmosphärischem Druck auf 0 bar überatmosphärischem Druck), um so ein Ausgasen von gegebenenfalls noch mit Kohlenoxiden gefüllten Katalysatorporen zu erleichtern. Dies kann in einfacher Weise mittels eines Druckhalteventils am Ausgang aus dem Reaktor erfolgen, das zu diesem Zweck in einfacher Weise entsprechend weiter geöffnet oder zur Rückeinstellung des Arbeitsdrucks wieder zunehmend geschlossen wird.

Ist ein im wesentlichen verschwindendes $\Delta G$ erreicht (zur Kontrolle kann man das Oxidationspotential des Regeneriergases noch geringfügig erhöhen und prüfen, ob $\Delta G$ dabei unverändert bleibt), ist der Dehydrierkatalysator von den Kohlenstoff enthaltenden Abscheidungen im wesentlichen wieder befreit. Die elementaren Konstituenten befinden sich wieder im reoxidierten Zustand und der Dehydrierkatalysator ist im Fall eines oxidischen Dehydrierkatalysators zum Zweck der heterogen katalysierten Dehydrierung wieder einsatzbereit. D.h., man wird abschließend die Eintrittstemperatur des Regeneriergases mit dem höchsten gewählten Oxidationspotential auf den Wert einstellen, der der Eintrittstemperatur des Reaktionsgases für die im Anschluss an die Regenerierung wieder aufgenommene heterogen katalysierte Dehydrierung entspricht und daran anschließend den Regeneriergasstrom abstellen und durch den entsprechenden Reaktionsgasstrom ersetzen.

Selbstredend kann man die erfindungsgemäße Regenerierung aber auch bereits beenden, bevor $\Delta G$ im wesentlichen den Wert Null erreicht. In diesem Fall erlangt die Regenerierung zwar nicht ihre maximal mögliche Ausprägung, von der Vorteilhaftigkeit der erfindungsgemäßen Verfahrensweise wird aber dennoch Gebrauch gemacht. Insbesondere unter dem Gesichtspunkt einer Gesamtwirtschaftlichkeitsbetrachtung kann es erfindungsgemäß zweckmäßig sein, die erfindungsgemäße Regenerierung bereits zu beenden, bevor $\Delta G$ im wesentlichen den Wert Null erreicht hat, und die heterogen katalysierte Dehydrierung vorzeitig wieder aufzunehmen. Im Zusammenspiel von Zeitaufwand und Dehydrierperformance kann das wirtschaftliche Optimum durchaus bei einer Teilregenerierung liegen (die Lage des wirtschaftlichen Optimums ist für das jeweilige Katalysatorsystem mittels weniger Einzelversuche zu ermitteln).

Handelt es sich beim verwendeten Dehydrierkatalysator dagegen um einen metallischen Dehydrierkatalysator, so beginnt sich dann, wenn mit dem das höchste Oxidationspotential aufweisenden Regeneriergas ein im wesentlichen verschwindendes $\Delta G$ erreicht ist, die sogenannte Redispergierung zu vervollständigen.

Wie bereits erwähnt, handelt es sich bei den metallischen Katalysatoren um solche Katalysatoren, die aus wenigstens einem auf einem, in der Regel oxidischen Träger abgeschiedenen Metall bestehen. Für eine hohe Aktivität eines solchen Katalysators ist es dabei von Vorteil, wenn die metallische Phase auf der Trägeroberfläche in möglichst feinteiliger Form dispers verteilt ist. Zu diesem Zweck wird bei der Herstellung eines solchen Katalysators normalerweise zunächst eine oxidierte Form des jeweiligen Metalls auf den Träger aufgebracht (z. B. in Form einer Salzlösung des entsprechenden Metalls durch entsprechende Imprägnierung des Trägers mit der Salzlösung). Aufgrund der attraktiven Wechselwirkung zwischen Metallsalz und z. B. oxidischem Träger ist das Metall nach erfolgter Imprägnierung auf der äußeren und inneren Trägeroberfläche besonders gleichmäßig verteilt. Nachfolgend wird das ionisch aufgebrachte Metall üblicherweise durch chemisches Reduzieren in seine elementare Form überführt, ohne dass der vorab der Reduktion erreichte Zerteilungsgrad des Metalls auf dem Träger nennenswert beeinträchtigt wird. Zur Deaktivierung eines metallischen Dehydrierkatalysators tragen nun im Verlauf des Langzeitbetriebs einer heterogen katalysierten partiellen Dehydrierung normalerweise zwei Phänomene bei. Zum einen die bereits beschriebene Abscheidung von Kohlenstoffen und/oder hochsiedenden Kohlenwasserstoffen, und zum anderen ein im Langzeitbetrieb einhergehender Verlust des Zerteilungsgrades der dispersen Metallverteilung auf dem Träger. Letzteres ist vermutlich darauf zurückzuführen, dass in der elementaren Form die Metall-Metall-Wechselwirkung die Metall-Träger-Wechselwirkung überragt, weshalb sich der ursprüngliche Zerteilungsgrad im Verlauf des Langzeitbetriebs einer heterogen katalysierten Dehydrierung rückentwickelt.

Im Rahmen des erfindungsgemäßen Regenerierverfahrens erfolgt nun nicht nur eine Eliminierung der erwähnten Kohlenstoff enthaltenden Ablagerungen. Vielmehr wird auch wenigstens eine Teilmenge des die katalytische Dehydrierwirkung bedingenden Metalls in eine oxidische Form umgewandelt. In der oxidischen Form erwächst aber wieder eine verstärkte Wechselwirkung mit dem Träger, aus welcher eine erwünschte Redispergierung erfolgt. Lässt man daher beim erfindungsgemäßen Verfahren auch noch dann, wenn ein $\Delta G$ von im wesentlichen Null erreicht ist, Regeneriergas mit dem höchsten Oxidationspotential auf den deaktivierten Metallkatalysator in erfindungsgemäßer Weise weiter einwirken, so fördert dies die vorbeschriebene Redispergierung. Man spricht daher auch von der Redispergierungsphase innerhalb des erfindungsgemäßen Regenerierverfahrens im Fall seiner Anwendung auf deaktivierte metallische Dehydrierkatalysatoren. Mit zunehmender Redispergierungsdauer nimmt die angestrebte Redispergierung in der Regel zu. In anwendungstechnisch typischer Weise wird die Redispergierungsphase normalerweise einen Zeitraum von wenigen Stunden (z.B. $\geq 2$ h, oder $\geq 6$ h) bis zu wenigen Tagen (z. B. $\leq 5$ Tage) in Anspruch nehmen. Die jeweils erreichte Redispergierungsgüte muss im Einzelfall jeweils mittels einer Probenahme dadurch experimentell überprüft werden, dass die Katalysatorprobe wieder als Dehydrierkatalysator eingesetzt wird. An dieser Stelle sei noch festgehalten, dass das Regeneriergas während der Redispergierphase erfindungsgemäß vorteilhaft keinen Wasserdampf enthält. Im Unterschied dazu wirkt sich bei der Reaktivierung von deaktivierten oxidischen Dehydrierkatalysatoren ein Beisein von Wasserdampf im Regeneriergas in der Regel über die gesamte erfindungsgemäße Regenerierungsstrecke vorteilhaft aus. Dies trifft bei deaktivierten metallischen Katalysatoren in der Regel auch auf die der Redispergierphase vorangehende Regenerierphase zu.

Derartige Wasserdampfgehalte des Regeneriergases sollten in der Regel jedoch 20 Vol.-% nicht übersteigen. Normalerweise werden solche Wasserdampfgehalte jedoch $\geq 1$ Vol.-% betragen.

Dabei ist zu berücksichtigen, dass bei der Oxidation von auf dem deaktivierten Dehydrierkatalysator abgeschiedenen schwersiedenden Kohlenwasserstoffen als Oxidationsprodukt auch Wasser gebildet wird.

Der erfindungsgemäß reoxidierte und in der Regel zusätzlich redispergierte metallische Dehydrierkatalysator ist jedoch im Unterschied zum erfindungsgemäß reoxidierten oxidischen Dehydrierkatalysator nicht unmittelbar als solcher für die Katalyse heterogen katalysierter partieller Dehydrierungen von zu dehydrierenden Kohlenwasserstoffen geeignet.

Vielmehr bedarf es dazu im Anschluss an die erfindungsgemäße Regenerierung noch einer Reduzierung der metallischen Konstituenten des redispergierten metallischen Dehydrierkatalysators in deren elementaren Zustand. Anwendungstechnisch vorteilhaft wird zu diesem Zweck molekularer Wasserstoff verwendet. Dieser wird zu diesem Zweck entweder als solcher oder in durch ein Inertgas verdünnter Form bei erhöhter Temperatur durch das Katalysatorbett geleitet. Vorzugsweise sollte der Wasserstoffgehalt eines solchen Reduziergases $\geq 1$ Vol.-%, vorzugsweise $\geq 3$ Vol.-% und besonders bevorzugt $\geq 5$ Vol.-% betragen.

Erfindungsgemäß vorteilhaft werden als solche Reduziergase Gasgemische eingesetzt, die wenigstens 40 Vol.-% (vorzugsweise 40 bis 60 Vol.-%) an molekularem Wasserstoff und als Restmenge im wesentlichen Inertgas enthalten. Als Inertgasbestandteile des Reduziergases eignen sich vor allem Ar, He, Ne, $N_2$ und $H_2O$, Anwendungstechnisch zweckmäßig werden als Inertgas im Reduziergas molekularer Stickstoff und/oder Wasserdampf verwendet. Die alleinige Verwendung von molekularem Stickstoff wird bevorzugt. Häufig kommen jedoch Reduziergase zum Einsatz, die neben Stickstoff als inertem Verdünnungsgas bis zu 5 Vol.-%, oder bis zu 3 Vol.-%, oder bis zu 1 Vol.-% an Wasserdampf enthalten. Dies ist nicht zuletzt dann der Fall, wenn das Reduziergas, wie bereits für das erfindungsgemäße Regeneriergas aufgezeigt, im Kreis gefahren wird. Eine solche Kreisfahrweise ist vor allem deshalb angebracht, weil das Reduziergas den molekularen Wasserstoff als Reduziermittel im Vergleich zum Oxidgehalt des redispergierten metallischen Dehydrierkatalysators in erheblichem Überschuss relativ zur Reaktionsstöchiometrie (Metalloxid + $H_2 \rightarrow$ $H_2O$ + Metall) aufweist (die oxidischen Träger sind normalerweise hochgebrannt (bei hoher Temperatur gebrannt) und werden durch die reduzierende Atmosphäre normalerweise nicht angegriffen). Aus entsprechenden hochgebrannten oxidischen Materialien sind üblicherweise die Inertformkörper zur Verdünnung des Katalysatorbetts gefertigt. Ihr spezifisches Porenvolumen beträgt in der Regel $\leq 0{,}5$ cm$^3$/g (vorzugsweise $\leq 0{,}25$ cm$^3$/g) und ihre spezifische Oberfläche $\leq$ 100 cm$^2$/g, vorzugsweise $\leq 70$ cm$^2$/g.

Darüber hinaus gewährleistet eine Kreisgasfahrweise auch in diesem Fall den Beibehalt des Wärmeinhalts des Reduziergases. Häufig muss noch nicht einmal frischer molekularer Wasserstoff ins im Kreis geführte Reduziergas ergänzt werden.

Die Thematik des Beibehalts des Wärmeinhalts ist nicht zuletzt deshalb relevant, weil die Reduzierung zum elementaren Metall gleichfalls erhöhte Temperaturen erfordert. Die mit der Reduzierung selbst einhergehende Wärmetönung ist vergleichsweise wenig ausgeprägt ("nur leicht exotherm").

Im Regelfall wird die Temperatur des Reduziergases (des reduzierend wirkenden Gases) beim Eintritt in das Katalysatorbett 350 bis 600°C, vorzugsweise 400 bis 550°C, oder 400 bis 500°C betragen. Besonders bevorzugt beträgt vorgenannte Eintrittstemperatur 430 bis 480°C. Das vorgenannte gilt insbesondere im Fall eines in einem adiabaten Reaktor befindlichen deaktivierten Katalysatorbetts.

Zu hohe Temperaturen im Katalysatorbett sind im Rahmen der vorgenannten Reduzierung zu vermeiden, da sie der im Rahmen der erfindungsgemäßen Regenerierung bewirkten Redispergierung gegebenenfalls entgegenwirken. Ebenso wie das Regeneriergas kann auch das Reduziergas grundsätzlich durch das Katalysatorbett gesaugt werden. In diesem Fall wird der Arbeitsdruck beim Reduziervorgang in der Regel unterhalb von Normaldruck liegen. Vorzugsweise befindet sich dieser Arbeitsdruck aber auch beim Reduziervorgang oberhalb vom Atmosphärendruck, wird auch das Reduziergas durch das Katalysatorbett gedrückt. Typische Arbeitsdrucke bewegen sich beim erfindungsgemäßen Reduziervorgang 1 bis 10 bar, bevorzugt 1 bis 6 bar und besonders bevorzugt 1 bis 3 bar oberhalb von Atmosphärendruck.

Im übrigen gilt für die Kreisgasfahrweise des Reduziergases das bei der Kreisgasfahrweise des erfindungsgemäßen Regeneriergases Gesagte. Beenden wird man den Reduziervorgang im wesentlichen dann, wenn der Gehalt des Reduziergases an Wasserdampf (ausgedrückt in Vol.-%) beim Austritt aus dem Katalysatorbett im Vergleich zum selben Gehalt beim Eintritt des Reduziergases in das redispergierte Katalysatorbett im wesentlichen nicht mehr erhöht ist.

Zum Abschluss der Reduzierung wird man die Temperatur des Reduziergases beim Eintritt desselben in das Katalysatorbett auf den Wert einstellen, der derjenigen Temperatur entspricht, die das Reaktionsgas bei der Wiederaufnahme des Dehydrierverfahrens nach beendeter Regenerierung des deaktivierten metallischen Dehydrierkatalysators aufweisen wird. Das Katalysatorbett wird dadurch auf diese Temperatur vorerwärmt. Anschließend wird von Reduziergas auf Reaktionsgas umgestellt.

An dieser Stelle sei nochmals festgehalten, dass alles in dieser Schrift Gesagte insbesondere dann gilt, wenn das Katalysatorbett ein Katalysatorfestbett ist und dies vor allem dann, wenn das Katalysatorbett (das Katalysatorfestbett) sich in einem adiabaten Reaktor befindet. Das Vorgenannte trifft vor allem dann zu, wenn die Deaktivierung im Rahmen einer von einer heterogen katalysierten Oxidehydrierung verschiedenen heterogen katalysierten Dehydrierung (z. B. einer solchen von Propan zu Propylen) erfolgt ist.

Unter einem Inertgas soll in dieser Schrift ein Gas verstanden werden, das zu $\geq$ 98,5 Vol.-% aus dem inerten Gas besteht. D.h., wird molekularer Stickstoff als Inertgas mitverwendet, so kann für diesen Zweck grundsätzlich industrieller Stickstoff mit einer Reinheit $\geq$ 98,5 Vol.-% verwendet werden (d.h., Stickstoff, der noch bis zu 1,5 Vol.-% an molekularem Sauerstoff enthalten kann). Die von Stickstoff (der inerten Komponente) und von molekularem Sauerstoff verschiedenen Bestandteile sollten dabei jedoch in ihrer Gesamtmenge auf $\leq$ 0,5 Vol.-% begrenzt sein.

Erfindungsgemäß bevorzugt wird natürlich Inertgas mit Reinheiten $\geq$99 Vol.-%, vorzugsweise $\geq$ 99,5 Vol.-% und besonders bevorzugt $\geq$99,9 Vol.-% verwendet.

In der Regel wird ein erfindungsgemäß zu regenerierendes deaktiviertes Deyhdrierkatalysatorbett, bezogen auf sein Gewicht (reine Inertmaterialschüttungen nicht miteingerechnet) 0,5 oder 1 bis 10 Gew.-% an darin abgeschiedenen Kohlenstoff enthaltenden Bestandteilen aufweisen (ausgedrückt als Gewichtsanteil des darin enthaltenen elementaren Kohlenstoffs). Insbesondere dann, wenn ein metallischer Dehydrierkatalysator verwendet wird und das für die heterogen katalysierte partielle Dehydrierung eingesetzte Reaktionsgasgemisch externen molekularen Wasserstoff sowie in der Regel Wasserdampf zugesetzt enthält, kann der Verlust des metallischen Zerteilungsgrades auch bereits ohne exorbitante Verkokung eine signifikante Deaktivierung des Katalysators bedingen (kann die Wirkung des geringeren Zerteilungsgrades die Wirkung der Verkokung dominieren).

In übrigen hat es sich vor der Inbetriebnahme des erfindungsgemäßen Regenerierverfahrens als zweckmäßig erwiesen, nach der Unterbrechung der zur Deaktivierung des Katalysatorbetts führenden heterogen katalysierten Dehydrierung das Katalysatorbett zunächst mit Inertgas (z.B. $N_2$, $H_2O$ und/oder Edelgase) zu spülen. Dadurch wird zum einen erreicht, dass das Katalysatorbett vorab des Beginns der erfindungsgemäßen Regenerierung von im Katalysatorbett noch enthaltenen, das erfindungsgemäße Regenerierverfahren gegebenenfalls störenden, Edukten und Produkten befreit wird. Zum anderen kann mit geeigneter Wahl der Eintrittstemperatur des Spülgases in das deaktivierte Katalysatorbett die Temperatur desselben, die am Ende eines Dehydrierzyklus in natürlicher Weise eine erhöhte ist, auf einen Wert vermindert werden, der mit einer vorteilhaften erfindungsgemäßen Regenerierung verträglicher ist (z.B. von über 550°C auf unter 450°C). Die Temperatur des diesbezüglich eingesetzten Spülgases wird dabei beim Eintritt in das Katalysator(fest)bett in der Regel im Bereich von $\geq$ 25°C bis $\leq$ 100°C liegen. Selbstverständlich kann das Spülgas dem Katalysatorbett aber auch auf eine über diesen Temperaturen liegende Temperatur erwärmt zugeführt werden. Diese Vorgehensweise wird man in der Regel dann wählen, wenn das Abkühlen des Katalysator(fest)betts nicht zu rasch erfolgen soll, um das Auftreten thermischer Spannungen im Dehydrierreaktor zu vermeiden. Als besonders günstiges Spülgas hat sich ein Gemisch aus molekularem Stickstoff und Wasserdampf erwiesen, das bis zu 5 Vol.-% Wasserdampf enthält.

Auch beim Spülen des Katalysatorbetts mit Spülgas ist es in der Regel zweckmäßig, wenigstens einen Teil des Spülgases im Kreis zu fahren. Dies trifft insbesondere dann zu, wenn alle das erfindungsgemäße Regenerierverfahren gegebenenfalls störenden Edukte und Produkte aus dem Katalysatorbett zuvor bereits herausgespült sind, die Temperatur des Katalysatorbetts aber noch nicht auf eine mit der vorteilhaften erfindungsgemäßen Regenerierung verträgliche Temperatur abgesenkt ist. Um den Bedarf an frischem Spülgas zu begrenzen, wird man in diesem Fall anwendungstechnisch zweckmäßig das aus dem Katalysatorbett erwärmt herausströmende Spülgas zunächst durch einen indirekten Wärmeaustauscher führen (z.B. einen Luftkühler), um seine Temperatur zunächst auf die gewünschte Temperatur zu senken, und es dann in das Katalysatorbett rückführen.

Wie bereits erwähnt, ist es erfindungsgemäß vorteilhaft, wenn die Belastung des Katalysatorbetts mit Regeneriergas im Verlauf des erfindungsgemäßen Regenerierverfahrens ein Maximum durchläuft. Bei Anwendung der diskutierten Kreisfahrweise des Regeneriergases bedeutet dies, dass am Anfang des erfindungsgemäßen Regenerierverfahrens zunehmend größere Regeneriergasmengen im Kreis zu fahren sind, wohingegen gegen Ende des erfindungsgemäßen Regenerierverfahrens die im Kreis zu führende Regeneriergasmenge nur noch eine vergleichsweise geringe ist. D.h., es wird somit für eine effiziente Durchführung des erfindungsgemäßen Verfahrens ein Radialgebläse benötigt, das sowohl große als auch geringe Kreisgasmengen zu fördern vermag. Als anwendungstechnisch vorteilhaft haben sich für diesen Zweck Radialgebläse erwiesen, die zwei voneinander verschiedene Antriebsmotoren mit voneinander verschiedener Leistungsstärke aufweisen (einen großen, leistungsstarken und einen kleinen, leistungsschwächeren). Der jeweils andere von beiden läuft im Leerlauf. Alternativ kann auch ein Antriebsmotor mit Frequenzumwandler eingesetzt werden.

Da das Katalysatorbett erfindungsgemäß bevorzugt ein Katalysatorfestbett ist, soll unter einem Dehydrierkatalysator in dieser Schrift im besonderen ein Formkörper verstanden werden, dessen Längstausdehnung L (längste direkte Verbindungslinie zweier auf der Oberfläche des Formkörpers befindlicher Punkte) 0,1 bzw. 1 bis 100 mm, oft 0,5 bis 80 mm oder 0,5 bis 50 mm, vorzugsweise 1 bis 30 mm oder 2 bis 20 mm und besonders bevorzugt 2 bis 10 mm bzw. 2 bis 5 mm beträgt (das Vorgenannte gilt auch für die Längstausdehnung von Inertformkörpern, mit denen Katalysatorformkörper im Katalysatorfestbett verdünnt werden können). Im Fall von für von heterogen katalysierten Oxidehydrierungen von zu dehydrierenden Kohlenwasserstoffen verschiedene heterogen katalysierte Dehydrierungen zu verwendende Dehydrierkatalysatoren, ist das erfindungsgemäße Verfahren insbesondere auf solche anwendbar, die im nachfolgend beschriebenen Versuch, bezogen auf einmaligen Durchgang des Reaktionsgasgemischs durch das Reaktionsrohr, wenigstens 5 mol-% des im Reaktionsgas enthaltenen Propan zu Propylen dehydrieren.

Ein Reaktionsrohr aus Stahl der EN Werkstoffnummer 1.4835 mit einer Wanddicke von 2 mm und einem Innendurchmesser von 35 mm sowie einer Länge von 80 cm wird wie folgt befüllt:

50 ml aus einer Schüttung des entsprechenden Dehydrierkatalysators werden im Reaktionsrohr mittig platziert. Oberhalb und unterhalb der Schüttung aus Katalysatorformkörper wird das Reaktionsrohr jeweils mit einer Schüttung aus Steatitkugeln (Inertkugeln) mit einem Kugeldurchmesser von 1,5 bis 2,5 mm aufgefüllt. Ein Gitterrost trägt die gesamte Schüttung. Von außen wird das Reaktionsrohr auf seiner gesamten Länge auf einer Temperatur von 550°C gehalten. Das Reaktionsrohr wird mit einem Gemisch aus Propan und Wasserdampf im Volumenverhältnis 2 (Propan) zur 1 (Wasserdampf) mit einer Propanbelastung der Schüttung aus Katalysatorformkörpern von 1000 Nl/l·h beschickt. Der in das Reaktionsrohr geführte Reaktionsgasgemischstrom ist auf eine Temperatur von 550°C vorerwärmt. Im besonderen eignet sich das erfindungsgemäße Verfahren für solche Dehydrierkatalysatoren, bei denen unter den vorstehenden Rahmenbedingungen die Summenselektivität der Bildung der Nebenprodukte Ethan, Ethylen und Methan ≤ 5 mol-%, bezogen auf umgesetztes Propan, beträgt.

Zu diesen Dehydrierkatalysatoren gehören z.B. diejenigen der deutschen Anmeldung Nr. 102005044916, insbesondere aber die metallischen Dehydrierkatalysatoren der DE-A 19937170, insbesondere jene gemäß Beispiel 1, Beispiel 2, Beispiel 3 und Beispiel 4 dieser Schrift.

Dabei handelt es sich um metallische Dehydrierkatalysatoren, die 10 bis 99,9 Gew.-% Zirkondioxid sowie 0 bis 60 Gew.-% Aluminiumoxid, Siliciumdioxid und/oder Titandioxid als Trägermaterial und in elementarer Form sowie in einer Gesamtmenge von 0,1 bis 10 Gew.-% wenigstens ein Element aus der Menge umfassend die erste und die zweite Hauptgruppe sowie die von Lanthan verschiedenen Elemente der dritten Nebengruppe, wenigstens ein Element der achten Nebengruppe (jeweils des Periodensystems der Elemente) sowie Lanthan und/oder Zinn, mit der Maßgabe enthalten, dass die Summe der Gewichtsprozente 100 Gew.-% ergibt.

Zu diesen metallischen Dehydrierkatalysatoren gehören als bevorzugte Ausführungsformen auch diejenigen, die 10 bis 99,9 Gew.-% Zirkondioxid sowie 0 bis 60 Gew.-% Aluminiumoxid, Siliciumdioxid und/oder Titandioxid als Trägermaterial und in elementarer Form sowie in einer Gesamtmenge von 0,1 bis 10 Gew.-% wenigstens ein Element der VIII. Nebengruppe, mindestens ein Element der I. und II. Hauptgruppe, mindestens ein Element der III. und/oder IV. Hauptgruppe und mindestens ein Element der III. Nebengruppe einschließlich Lanthaniden und Acteniden mit der Maßgabe enthalten, dass die Summe der Gewichtsprozente 100 Gew.-% ergibt.

Als Element der VIII. Nebengruppe enthält die Aktivmasse der vorgenannten metallischen Dehydrierkatalysatoren bevorzugt Platin und/oder Palladium, besonders bevorzugt Platin. Als Element der I. und II. Hauptgruppe enthält die Aktivmasse der vorgenannten Dehydrierkatalysatoren bevorzugt Kalium und/oder Cäsium. Als Element der III. Nebengruppe einschließlich der Lanthaniden und Actiniden enthält die Atkivmasse der vorgenannten Dehydrierkatalysatoren bevorzugt Lanthan und/oder Cer. Als Elemente der III. und/oder IV. Hauptgruppe enthält die Aktivmasse der vorgenannten Dehydrierkatalysatoren bevorzugt ein oder mehrere Elemente aus der Gruppe bestehend aus Bor, Gallium, Silizium, Germanium, Indium, Zinn und Blei, besonders bevorzugt Zinn.

Grundsätzlich bestehen hinsichtlich der Anwendbarkeit der erfindungsgemäßen Verfahrensweise auf ein deaktiviertes Katalysatorfestbett weder hinsichtlich der Katalysatorgeometrie (insbesondere im Fall von Trägerkatalysatoren) noch hinsichtlich der Geometrie von zur Verdünnung des Katalysatorfestbetts gegebenenfalls mitverwendeten inerten Formkörpern irgendwelche Einschränkungen. Besonders häufig angewandte Geometrien sind z.B. Vollzylinder, Hohlzylinder (Ringe), Kugeln, Kegel, Pyramiden und Würfel sowie Stränge, Wagenräder, Sterne und Monolithe.

Insbesondere im Fall von von heterogen katalysierten partiellen Oxidehydrierungen verschiedenen heterogen katalysierten partiellen Dehydrierungen (das sind in dieser Schrift die nichtoxidativen und die oxidativen konventionellen heterogen katalysierten partiellen Dehydrierungen) kommen als Geometrien für ein Katalysatorfestbett in Betracht: Katalysatorstränglinge (Durchmesser typisch 0,1 bzw. 1 bis 10 mm, bevorzugt 1,5 bis 5 mm; Länge typisch 1 bis 20 mm, bevorzugt 3 bis 10 mm); Tabletten (vorzugsweise gleiche Abmessungen wie bei den Strängen) und/oder Katalysatorringe (Außendurchmesser und Länge jeweils typisch 2 bis 30 mm oder bis 10 mm, Wandstärke zweckmäßig 1 bis 10 mm, oder bis 5 mm, oder bis 3 mm). Diese Geometrien kommen grundsätzlich auch als Geometrien für gegebenenfalls mitverwendete Inertformkörper zur Verdünnung des Katalysatorfestbetts in Betracht.

In der Regel sind die Dehydrierkatalysatoren (insbesondere die metallischen Dehydrierkatalysatoren und unter diesen insbesondere die in der DE-A 19937107 empfohlenen (insbesondere die beispielhaften Katalysatoren dieser DE-A)), die sich für oxidative und nicht oxidative konventionelle heterogen katalysierte partielle Dehydrierungen eignen, so beschaffen, dass sie sowohl die Dehydrierung des zu dehydrierenden Kohlenwasserstoffs (z.B. Propan) als auch die Verbrennung des zu dehydrierenden Kohlenwasserstoffs (z.B. Propan) und die Verbrennung von molekularem Wasserstoff zu katalysieren vermögen. Die Wasserstoffverbrennung verläuft dabei sowohl in Vergleich zur Dehydrierung des zu dehydrierenden Kohlenwasserstoffs (z.B. Propans) als auch im Vergleich zu dessen Verbrennung im Fall einer Konkurrenzsituation an den Katalysatoren sehr viel schneller.

Zur Durchführung einer der erfindungsgemäßen Regenerierung vorausgehenden, die Deaktivierung des erfindungsgemäß zu regenerierenden Katalysatorbetts bedingenden, konventionellen (oxidativen oder nicht oxidativen) heterogen katalysierten partiellen Dehydrierung eines zu dehydrierenden Kohlenwasserstoffs (z.B. Propan) kommen prinzipiell alle im Stand der Technik bekannten Reaktortypen und Verfahrensvarianten in Betracht.

Beschreibungen solcher Verfahrensvarianten enthalten zum Beispiel alle bezüglich der für solche Dehydrierungen geeigneten Dehydrierkatalysatoren in dieser Schrift zitierten Schriften des Standes der Technik sowie der eingangs dieser Schrift zitierte Stand der Technik und die Schriften DE-A 10 2006 029 790, DE-A 10 2006 035 718, DE-A 10 2006 0017 623, DE-A 10 2006 015 235 sowie DE-A 10 2005 061 626. Das gleiche gilt für die Schriften EP-A 1109763, US-A 3,308,181, US-A 3, 670,044, US-A 4,886,928, US-A 6,566,573, US-A 4,788,371 und WO 94/29021, sowie den in diesen Schriften zitierten Stand der Technik.

Eine vergleichsweise ausführliche Beschreibung solcher konventioneller heterogen katalysierter partieller Dehydrierungen (nicht oxidativ oder oxidativ) enthält z.B. Catalytica® Studies Division, Oxidative Dehydrogenation and Alternative Dehydrogenation Processes, Study Number 4192 OD, 1993, 430 Ferguson Drive, Mountain View, California, 94043-5272, U.S.A..

Charakteristisch für eine konventionelle partielle heterogen katalysierte Dehydrierung von zu dehydrierenden Kohlenwasserstoffen (z.B. Propan) ist (wie bereits gesagt), dass der Dehydrierschritt endotherm verläuft. Das heißt, die für die Einstellung der erforderlichen Reaktionstemperatur benötigte Wärme (Energie) muss entweder dem Reaktionsgas vorab und/oder im Verlauf der heterogen katalysierten Dehydrierung zugeführt werden.

D. h., bezogen auf den einmaligen Durchgang des den zu dehydrierenden Kohlenwasserstoff enthaltenden Reaktionsgasgemischs durch das Katalysatorbett kann der das Katalysatorbett enthaltende Reaktionsraum durch gezielten Wärmeaustausch mit z. B. außerhalb des von einer Einhüllenden umschlossenen Reaktionsraums geführten fluiden (d.h. flüssigen oder gasförmigen) Wärmeträgern isotherm gestaltet werden (extern gelenkter Temperaturverlauf). Entsprechende Wärmeaustauscher können aber auch im Reaktionsraum selbst untergebracht sein.

Eine konventionelle partielle heterogen katalysierte Dehydrierung von zu dehydrierenden Kohlenwasserstoffen (z.B. Propan) kann mit gleicher Bezugsbasis aber auch adiabat, d.h. im wesentlichen ohne einen solchen gezielten Wärmeaustausch mit (extern) geführten Wärmeträgern ausgeführt werden (extern ungelenkter Temperaturverlauf). Im letzteren Fall kann die auf den einmaligen Durchgang durch das im Reaktionsraum befindliche Katalysatorbett bezogene Bruttowärmetönung durch im folgenden noch zu beschreibenden intern gelenkten (z. B. durch Wasserstoffverbrennung in einem Folgeschritt) Temperaturverlauf sowohl endotherm (negativ), oder autotherm (im wesentlichen Null) oder exotherm (positiv) gestaltet werden.

In typischer Weise benötigt eine konventionelle heterogen katalysierte partielle Dehydrierung wenigstens eines zu dehydrierenden Kohlenwasserstoffs (z. B. von Propan), wie bereits erwähnt, vergleichsweise hohe Reaktionstemperaturen. Der dabei erzielbare Umsatz ist normalerweise durch das thermodynamische Gleichgewicht begrenzt. Typische Reaktionstemperaturen betragen 300 bis 800°C, bzw. 400 bis 700°C. Pro Molekül an z. B. zu Propylen dehydriertem Propan wird dabei ein Molekül Wasserstoff erzeugt. Hohe Temperaturen und Entfernung des Reaktionsproduktes $H_2$ begünstigen die Gleichgewichtslage im Sinne des Zielprodukts ebenso wie Partialdruckerniedrigung durch inerte Verdünnung. Die zugehörigen Arbeitsdrucke betragen in der Regel 0,2 bis 10 bar, bzw. 0,5 bis 6 bar oder 0,5 bis 3 bar (jeweils absolut).

Ferner ist es, wie bereits gesagt, für konventionelle heterogen katalysierte partielle Dehydrierungen wenigstens eines zu dehydrierenden Kohlenwasserstoffs (z. B. von Propan) aufgrund der hohen benötigten Reaktionstemperaturen typisch, dass in gewissen Mengen schwersiedende hochmolekulare organische Verbindungen, bis hin zum Kohlenstoff, gebildet werden, die sich auf der Katalysatoroberfläche abscheiden und selbige dadurch deaktivieren. Um diese nachteilige Begleiterscheinung zu minimieren, kann das zur konventionellen heterogen katalysierten Dehydrierung bei erhöhter Temperatur über die Katalysatoroberfläche zu leitende, den zu dehydrierenden Kohlenwasserstoff (z. B. das Propan) enthaltende Reaktionsgas mit Wasserdampf verdünnt werden. Sich abscheidender Kohlenstoff wird unter den so gegebenen Bedingungen nach dem Prinzip der Kohlevergasung kontinuierlich teilweise wieder eliminiert.

Eine gewisse Unterdrückung der Bildung von Kohlenstoffablagerungen (und damit eine Verlängerung der Katalysatorstandzeit (Betriebsdauer einer heterogen katalysierten partiellen Dehydrierung zwischen zwei Regenerierungen des Katalysatorbetts)) ist aber auch dadurch möglich, dass man dem konventionell heterogen katalysiert zu dehydrierenden Kohlenwasserstoff (z. B. Propan), bevor er bei erhöhter Temperatur über den Dehydrierkatalysator geführt wird, molekularen Wasserstoff zusetzt.

Selbstverständlich besteht auch die Möglichkeit, dem konventionell heterogen katalysiert zu dehydrierenden Kohlenwasserstoff (z. B. Propan), insbesondere zu vorgenannten Zwecken, Wasserdampf und molekularen Wasserstoff im Gemisch zuzusetzen. Ein Zusatz von molekularem Wasserstoff zur konventionellen heterogen katalysierten Dehydrierung von Propan mindert auch die unerwünschte Bildung von Allen (Propadien), Propin und Acetylen als Nebenprodukten.

Es kann nun (wie bereits angesprochen) zweckmäßig sein, die konventionelle heterogen katalysierte Kohlenwasserstoff(z. B. Propan)dehydrierung (z. B. mit vergleichsweise geringem Propan(bzw. generell Kohlenwasserstoff)umsatz) (quasi) adiabat durchzuführen. Das heißt, man wird das der Dehydrierung zuzuführende Reaktionsgasgemisch (in dieser Schrift auch als Ausgangsgas oder Ausgangsgasgemisch bezeichnet) in der Regel zunächst auf eine Temperatur von 400 bzw. 500 bis 700°C (beziehungsweise von 550 bis 650°C) erhitzen (zum Beispiel durch Direktbefeuerung der es umgebenden Wandung). Im Normalfall wird dann ein einziger adiabater Durchgang durch wenigstens ein im adiabat gestalteten Reaktionsraum befindliches Katalysatorbett ausreichend sein, um den gewünschten Umsatz zu erzielen, wobei sich das Reaktionsgas um etwa 30°C bis 200°C (je nach Umsatz und Verdünnung) abkühlen wird. Ein Beisein von Wasserdampf als Wärmeträger macht sich auch unter dem Gesichtspunkt einer adiabaten Fahrweise vorteilhaft bemerkbar. Die vergleichsweise niedrige Reaktionstemperatur ermöglicht längere Standzeiten des verwendeten Katalysatorbetts.

Prinzipiell ist eine konventionelle heterogen katalysierte Kohlenwasserstoff(z. B. Propan)dehydrierung (unabhängig ob adiabat oder isotherm gefahren) sowohl im Katalysatorfestbett, als auch in einem Wanderbett oder Wirbelbett durchführbar. Die Durchführung im Katalysatorfestbett ist normalerweise bevorzugt. Alle Aussagen in dieser Schrift beziehen sich daher insbesondere auf eine Durchführung im Katalysatorfestbett.

Als Katalysatorbeschickung für eine konventionelle heterogen katalysierte Dehydrierung eines zu dehydrierenden Kohlenwasserstoffs (z. B. Propan) mit einem wie beschrieben vergleichsweise geringen Umsatz bei einmaligem Durchgang durch das im jeweiligen Reaktionsraum befindliche Katalysatorbett, eignen sich alle in dieser Schrift bezüglich einer solchen Dehydrierung als geeignet offenbaren, vor allem beispielhaft offenbaren, Katalysatoren, sowie deren Gemische mit bezüglich der konventionellen heterogen katalysierten Dehydrierung inerten geometrischen Formkörpern.

Eine konventionelle heterogen katalysierte Kohlenwasserstoff(z. B. Propan)dehydrierung kann bei Katalysatorbelastungen (bezogen auf die eingesetzte Katalysatorgesamtmenge) sowohl mit Ausgangsgas als auch mit darin enthaltenem zu dehydrierendem Kohlenwasserstoff (z. B. Propan) von 100 bis 10000 h$^{-1}$, häufig 300 bis 5000 h$^{-1}$, das heißt vielfach 500 bis 3000 h$^{-1}$ sowohl bei geringem (z.B. ≤ 30 mol-%, oder ≤ 20 mol-%, oder ≤ 15 mol-%) als auch bei hohem (≥ 30 mol-%) Umsatz an zu dehydrierendem Kohlenwasserstoff (z. B. Propan) betrieben werden.

In besonders eleganter Weise lässt sich eine konventionelle heterogen katalysierte Dehydrierung wenigstens eines zu dehydrierenden Kohlenwasserstoffs (z. B. Propan) (sowohl bei Dehydrierumsätzen ≤ 30 mol-%, oder ≤ 20 mol-%, oder ≤ 15 mol-% als auch > 30 mol-% (z. B. 40 mol-%, oder 50 mol-%)) in einem Hordenreaktionsraum verwirklichen.

Ein solcher enthält räumlich aufeinanderfolgend mehr als ein die Dehydrierung katalysierendes Katalysatorbett. Die Katalysatorbettenzahl kann z. B. 1 bis 20, zweckmäßig 2 bis 8, aber auch 3 bis 6 betragen. Vorzugsweise sind die Katalysatorbetten radial oder axial hintereinander angeordnet. Anwendungstechnisch zweckmäßig wird in einem solchen Hordenreaktionsraum der Katalysatorfestbetttyp angewendet.

Im einfachsten Fall sind die Katalysatorfestbetten im Reaktionsraum axial oder in den Ringspalten von zentrisch ineinandergestellten zylindrischen Gitterrosten angeordnet. Es ist jedoch auch möglich, die Ringspalte im Reaktionsraum in Segmenten übereinander anzuordnen und das Gas nach radialem Durchtritt in einem Segment in das nächste darüber- oder darunter liegende Segment zu führen.

In zweckmäßiger Weise wird das Reaktionsgas (Ausgangsgas) auf seinem Weg von einem Katalysatorbett zum nächsten Katalysatorbett zum Beispiel durch Überleiten über mit heißen Gasen erhitzte Wärmetauscherrippen oder durch Leiten durch mit heißen Brenngasen erhitzte Rohre bzw. mit heißen Gasen erhitzte Wärmetauscherplatten, im Hordenreaktionsraum einer Zwischenerhitzung unterworfen.

Wird das erfindungsgemäße Verfahren im Hordenreaktionsraum im übrigen adiabat betrieben, ist es für Dehydrierumsätze (z. B. Propanumsätze) ≤ 50 mol-%, oder ≤ 40 mol-%, oder ≤ 30 mol-%, insbesondere bei Verwendung der in der DE-A 199 37 107 beschriebenen Katalysatoren, insbesondere der beispielhaften Ausführungsformen, ausreichend, das Ausgangsgas auf eine Temperatur von 400 bzw. 450 bis 550°C (vorzugsweise 400 bis 500°C) vorerhitzt in den Reaktionsraum zu führen und innerhalb des Hordenreaktionsraums wenigstens in diesem Temperaturbereich zu halten. Das heißt, die gesamte konventionelle Dehydrierung ist so, wenigstens mit frischen Katalysatoren, bei vergleichsweise gemäßigten Temperaturen zu verwirklichen, was sich für die Standzeit der Katalysatorfestbetten zwischen zwei Regenerierungen als besonders günstig erweist.

Noch geschickter ist es, eine konventionelle heterogen katalysierte Dehydrierung im beschriebenen Hordenreaktor (wie ebenfalls bereits angesprochen) im wesentlichen autotherm durchzuführen, d. h., die vorstehend geschilderte Zwischenerhitzung auf direktem Weg durchzuführen (autotherme Fahrweise).

Dazu kann dem Reaktionsgas auf seinem Weg durch die Katalysatorfestbetthorden z.B. nach Durchströmung des ersten Katalysatorbettes und zwischen den nachfolgenden Katalysatorbetten vorteilhaft in begrenztem Umfang molekularer Sauerstoff zugesetzt werden. Je nach verwendetem Dehydrierkatalysator wird so eine begrenzte Verbrennung der im Reaktionsgas enthaltenen Kohlenwasserstoffe, gegebenenfalls bereits auf der Katalysatoroberfläche abgeschiedener Kohle beziehungsweise kohleähnlicher Verbindungen und/oder von im Verlauf der konventionellen heterogen katalysierten Dehydrierung (z. B. einer Propandehydrierung) gebildetem und/oder dem Reaktionsgas zugesetztem Wasserstoff bewirkt (es kann auch anwendungstechnisch zweckmäßig sein, im Hordenreaktionsraum Katalysatorbetten einzufügen, die mit Katalysator beschickt sind, der spezifisch (selektiv) die Verbrennung von Wasserstoff (und/oder von Kohlenwasserstoff) katalysiert (als solche Katalysatoren kommen zum Beispiel jene der Schriften US-A 4,788,371, US-A 4,886,928, US-A 5,430,209, US-A 5,530,171, US-A 5,527,979 und US-A 5,563,314 in Betracht; beispielsweise können solche Katalysatorbetten in alternierender Weise zu den Dehydrierkatalysator enthaltenden Betten im Hordenreaktionsraum untergebracht sein)). Die dabei freigesetzte Reaktionswärme ermöglicht so (quasi adiabate Reaktorgestaltung) auf quasi autotherme Weise eine nahezu isotherme (interne Temperaturlenkung) Betriebsweise der heterogen katalysierten (z. B. Propan)Dehydrierung. Mit zunehmender gewählter Verweilzeit des Reaktionsgases im Katalysatorbett ist so eine (z. B. Propan)Dehydrierung bei abnehmender oder im wesentlichen konstanter Temperatur möglich, was besonders lange Standzeiten zwischen zwei Regenerierungen ermöglicht (anstelle eines Hordenreaktors kann in dazu äquivalenter Weise auch eine Hintereinanderschaltung (Reihenschaltung) von einer der vorgenannten Hordenanzahl entsprechenden Anzahl von Reaktoren angewendet und dabei die Sauerstoffeinspeisung jeweils zwischen aufeinanderfolgenden Reaktoren vorgenommen werden. Die Anzahl von so verschalteten Reaktoren kann z. B. "drei", oder "zwei", oder "vier" betragen. In der Regel wird jeder einzelne dieser Reaktoren ein bzw. höchstens zwei Katalysatorfestbetten aufweisen).

Durch eine wie beschrieben durchgeführte Folgeverbrennung von im Verlauf der Dehydrierung gebildetem molekularem Wasserstoff wird aus einer nichtoxidativen konventionellen heterogen katalysierten Dehydrierung eine im Sinne der vorliegenden Anmeldung oxidative konventionelle heterogen katalysierte Dehydrierung.

In der Regel sollte eine wie vorstehend beschriebene Sauerstoffeinspeisung so vorgenommen werden, dass der Sauerstoffgehalt des Reaktionsgases, bezogen auf die darin enthaltene Menge an zu dehydrierendem Kohlenwasserstoff und dehydriertem Kohlenwasserstoff (z. B. Propan und Propylen), 0,01 bzw. 0,5 bis 30 Vol.-% beträgt. Als Sauerstoffquelle kommen dabei sowohl reiner molekularer Sauerstoff oder mit Inertgas, zum Beispiel $CO$, $CO_2$, $N_2$, Edelgase, verdünnter Sauerstoff, insbesondere aber auch Luft, in Betracht. Eine alternative Sauerstoffquelle können Stickoxide sein. Die resultierenden Verbrennungsgase wirken in der Regel zusätzlich verdünnend und fördern dadurch die konventionelle heterogen katalysierte (z. B. Propan) Dehydrierung.

Die Isothermie einer konventionellen heterogen katalysierten (z. B. Propan)Dehydrierung lässt sich dadurch weiter verbessern, dass man im die Katalysatorbetthorden enthaltenden Reaktionsraum in den Räumen zwischen den Katalysatorbetten geschlossene, vor ihrer Füllung günstigerweise aber nicht notwendigerweise evakuierte, Einbauten (zum Beispiel rohrförmige), anbringt. Derartige Einbauten können auch ins jeweilige Katalysatorbett gestellt werden. Diese Einbauten enthalten geeignete Feststoffe oder Flüssigkeiten, die oberhalb einer bestimmten Temperatur verdampfen oder schmelzen und dabei Wärme verbrauchen und dort, wo diese Temperatur unterschritten wird, wieder kondensieren und dabei Wärme freisetzen.

Eine Möglichkeit, das Ausgangsgas bzw. den Ausgangsgasstrom für eine konventionelle heterogen katalysierte (z. B. Propan)Dehydrierung im das die Dehydrierung katalysierende Katalysatorbett enthaltenden Reaktionsraum auf die benötigte Reaktionstemperatur zu erwärmen, besteht auch darin, einen Teil des darin enthaltenen zu dehydrierenden Kohlenwasserstoffs (z. B. des Propans) und/oder $H_2$ mittels im Ausgangsgas enthaltenem molekularem Sauerstoff beim Eintritt in den Reaktionsraum zu verbrennen (zum Beispiel an geeigneten spezifisch wirkenden Verbrennungskatalysatoren, zum Beispiel durch einfaches Überleiten und/oder Durchleiten) und mittels der so freigesetzten Verbrennungswärme die Erwärmung auf die (für die Dehydrierung) gewünschte Reaktionstemperatur zu bewirken. Die resultierenden Verbrennungsprodukte, wie $CO_2$, $H_2O$ sowie das den für die Verbrennung benötigten molekularen Sauerstoff gegebenenfalls begleitende $N_2$ bilden vorteilhafte inerte Verdünnungsgase.

Besonders elegant lässt sich die vorgenannte Wasserstoffverbrennung wie in der WO 03/076370 bzw. DE-A 102 11 275 beschrieben realisieren. D.h., in einem Verfahren der kontinuierlichen konventionellen oxidativen heterogen katalysierten partiellen Dehydrierung von zu dehydrierendem Kohlenwasserstoff (z. B. im Propan) an wenigstens einem in einem Reaktionsraum befindlichen Katalysatorbett, bei dem man

- dem Reaktionsraum wenigstens einen, wenigstens einen zu dehydrierenden Kohlenwasserstoff (z. B. Propan), molekularen Sauerstoff, molekularen Wasserstoff und gegebenenfalls Wasserdampf enthaltenden Ausgangsgasstrom kontinuierlich zuführt,

- im Reaktionsraum den wenigstens einen zu dehydrierenden Kohlenwasserstoff durch wenigstens eine im Reaktionsraum befindliche Katalysatorbett führt, an welchem durch konventionelle heterogen katalysierte Dehydrierung molekularer Wasserstoff und wenigstens partiell wenigstens ein dehydrierter Kohlenwasserstoff (z. B. Propylen) gebildet werden,

- dem Reaktionsgas nach seinem Eintritt in den erfindungsgemäßen Reaktionsraum auf seinem Weg durch den Reaktionsraum gegebenenfalls weiteres molekularen Sauerstoff enthaltendes Gas zusetzt,

- der molekulare Sauerstoff im Reaktionsraum im Reaktionsgas enthaltenen molekularen Wasserstoff wenigstens teilweise zu Wasserdampf oxidiert, und

- dem Reaktionsraum wenigstens einen Produktgasstrom kontinuierlich entnimmt, der molekularen Wasserstoff, Wasserdampf, dehydrierten Kohlenwasserstoff (z. B. Propylen) und zu dehydrierenden Kohlenwasserstoff (z. B. Propan) enthält,

das dadurch gekennzeichnet ist, dass der dem Reaktionsraum entnommene wenigstens eine Produktgasstrom in zwei Teilmengen identischer Zusammensetzung aufgeteilt und eine der beiden Teilmengen als Dehydrierkreisgas in den dem erfindungsgemäßen Reaktionsraum zugeführten wenigstens einen Ausgangsgasstrom rückgeführt und die andere Teilmenge anderweitig weiterverwendet (z. B. zum Zweck einer heterogen katalysierten partiellen Oxidation von im Reaktionsraum gebildetem dehydriertem Kohlenwasserstoff) wird.

An dieser Stelle sei noch festgehalten, dass der Ausgangsgasstrom (der dem die Dehydrierung katalysierenden Katalysatorbett zugeführte Reaktionsgasgemischstrom) einer oxidativen oder nicht oxidativen konventionellen heterogen katalysierten partiellen Dehydrierung eines zu dehydrierenden Kohlenwasserstoffs in der Regel $\geq$ 5 Vol.-% des zu dehydrierenden Kohlenwasserstoffs (z.B. Propan) enthält. Häufig liegt dieser Volumenanteil bei entsprechend bezogenen Werten von $\geq$ 10 Vol.-%, oft $\geq$ 15 Vol.-%, und meist $\geq$ 20 Vol.-%, bzw. $\geq$ 25 Vol.-%, oder $\geq$ 30 Vol.-%. In der Regel liegt dieser Volumenanteil jedoch bei in gleicher Weise bezogenen Werten von $\leq$ 90 Vol.-%, meist $\leq$ 80 Vol.-% und oft $\leq$ 70 Vol.-%. Vorstehende Angaben gelten insbesondere im Fall von Propan als zu dehydrierendem Kohlenwasserstoff und Propylen als dehydriertem Kohlenwasserstoff. Selbstredend treffen sie aber auch dann zu, wenn iso-Butan der zu deyhdrierende Kohlenwasserstoff und iso-Buten der dehydrierte Kohlenwasserstoff ist. Daneben kann er (der Ausgangsgasstrom) z.B. enthalten:

a) $N_2$ und $H_2O$;
b) $N_2$, $O_2$ und $H_2O$;
c) $N_2$, $O_2$, $H_2O$ und $H_2$;
d) $N_2$, $O_2$, $H_2O$, $H_2$ und $CO_2$;
e) $N_2$, $O_2$, $H_2O$, $H_2$, $CO_2$ und CO.

Im Fall einer oxidativen konventionellen heterogen katalysierten partiellen Dehydrierung eines zu dehydrierenden Kohlenwasserstoffs können als Sauerstoffquelle sowohl reiner molekularer Sauerstoff, Luft oder sonstige Mischungen aus molekularem Sauerstoff und Inertgas verwendet werden.

Der mit zunehmender Betriebsdauer einer konventionellen partiellen heterogen katalysierten Dehydrierung eines zu dehydrierenden Kohlenwasserstoffs einhergehenden zunehmenden Deaktivierung des Katalysatorbetts kann insbesondere wie in der DE-A 10 2006 035 718 beschrieben entgegengewirkt werden (z.B. Erhöhung der Reaktionstemperatur durch interne und/oder externe Temperaturlenkung).

Mit den diesbezüglich zu ergreifenden Maßnahmen geht in der Regel jedoch auch eine Zunahme der unerwünschten Nebenproduktbildung einher, weshalb nach einer bestimmten Betriebsdauer die regenerierende Anwendung der erfindungsgemäßen Verfahrensweise von Vorteil ist.

Wurde die heterogen katalysierte partielle Dehydrierung in einem Hordenreaktor durchgeführt, der in Strömungsrichtung des Reaktionsgases eine Abfolge von mehreren räumlich aufeinanderfolgenden Katalysatorbetten (Katalysatorbetthorden) enthält, so kann die erfindungsgemäße Regenerierung des Katalysatorgesamt(fest)betts grundsätzlich auf unterschiedliche Art und Weise erfolgen.

Zum einen kann die Regenerierung des Katalysatorgesamtbetts (das ist die Gesamtheit aller im Hordenreaktor befindlichen einzelnen Katalysatorbetten) als solchem vorgenommen werden. D.h., man führt dem Hordenreaktor das Regeneriergas mit der entsprechenden Temperatur zu, und führt es innerhalb des Hordenreaktors entsprechend der räumlichen Aufeinanderfolge der einzelnen Katalysatorbetten sukzessive durch jedes einzelne Katalysatorbett. Die Regenerierung des Katalysatorgesamtbetts erfolgt dann in völliger Analogie zur Regenerierung eines einzelnen deaktivierten Katalysatorbetts (die Summe der Einzelbetten bildet faktisch das zu regenerierende Katalysatorbett).

Grundsätzlich besteht bei einem Hordenreaktor aber auch die Möglichkeit, jedes einzelne Katalysatorbett, jede einzelne Katalysatorbetthorde für sich in erfindungsgemäßer Weise zu regenerieren. Anwendungstechnisch zweckmäßig wird man dabei so vorgehen, dass man mit der Regenerierung des in Strömungsrichtung letzten Katalysatorbetts (der in Strömungsrichtung letzten Katalysatorbetthorde) beginnt. D.h., man führt das Regeneriergas zwischen dem in Strömungsrichtung letzten und vorletzten Katalysatorbett zu, und leitet es ausschließlich durch das letzte Katalysatorbett. Nach erfolgter Regenerierung des in Strömungsrichtung letzten Katalysatorbetts wird die Regenerierung mit der Regenerierung des in Strömungsrichtung vorletzten Katalysatorbetts fortgesetzt usw.. D.h., das Regeneriergas wird dann zwischen dem in Strömungsrichtung drittletzten und dem in Strömungsrichtung vorletzten Katalysatorbett zugeführt und zunächst durch das in Strömungsrichtung vorletzte und aus diesem ausströmend durch das in Strömungsrichtung letzte Katalysatorbett geführt usw.. In völliger Entsprechung können auch die auf eine Hintereinanderschaltung von verschiedenen Dehydrierreaktoren verteilten, die Dehydrierung katalysierenden, Katalysatorbetten erfindungsgemäß regeneriert werden. Selbstverständlich können aber in verschiedenen Dehydrierreaktoren befindliche Katalysatorbetten (unabhängig von deren Verschaltung) auch voneinander unabhängig und zeitgleich erfindungsgemäß regeneriert werden.

Zur Durchführung einer konventionellen (oxidativen oder nichtoxidativen) heterogen katalysierten Dehydrierung, insbesondere für eine adiabate Betriebsweise, ist der Innenraum eines einfachen Schachtofens ("Schachtofenreaktor") als das wenigstens eine Katalysatorbett (z. B. das wenigstens eine Katalysatorfestbett) enthaltender Reaktionsraum ausreichend, der vom Ausgangsgasstrom axial und/oder radial durchströmt wird. Eine besonders bevorzugte Ausführungsform eines solchen Reaktors beschreiben die Deutschen Aktenzeichen DE-A 10 2006 029 790, DE-A 10 2006 035 718, DE-A 10 2006 017 623.5 sowie DE-A 10 2006 015 235.2.

Im einfachsten Fall handelt es sich dabei zum Beispiel um einen im wesentlichen zylindrischen Behälter, dessen Innendurchmesser 0,1 bis 10 m, eventuell 0,5 bis 5 m beträgt und in welchem das wenigstens eine Katalysatorfestbett auf einer Trägervorrichtung (zum Beispiel ein Gitterrost) aufgebracht ist. Der mit Katalysator beschickte Reaktionsraum, dessen erfindungsgemäße Einhüllende im adiabaten Betrieb gegenüber ihrer Umgebung durch Aufbringen von entsprechenden Isoliermaterialien (z. B. Glaswolle) zusätzlich wärmeisoliert ist, wird dabei zweckmäßig mit dem heißen, den zu dehydrierenden Kohlenwasserstoff (z. B. das Propan) enthaltenden Ausgangsgasstrom axial durchströmt. Die Katalysatorgeometrie kann dabei sowohl kugelförmig als auch ringförmig oder strangförmig sein. Da im ebenda beschriebenen Fall der Reaktionsraum durch einen sehr kostengünstigen Apparat zu realisieren ist, sind alle Katalysatorgeometrien, die einen besonders niedrigen Druckverlust aufweisen, zu bevorzugen. Das sind vor allem Katalysatorgeometrien, die zu einem großen Hohlraumvolumen führen oder strukturiert aufgebaut sind, wie zum Beispiel Monolithe bzw. Wabenkörper. Zur Verwirklichung einer radialen Strömung des den zu dehydrierenden Kohlenwasserstoff (z. B. das Propan) enthaltenden Reaktionsgases kann der erfindungsgemäße Reaktionsraum zum Beispiel zwei konzentrisch ineinander gestellte zylindrische Gitterroste enthalten und die Katalysatorschüttung in deren Ringspalt angeordnet sein. Im adiabaten Fall wäre die ihn umgebende Einhüllende (die Mantelhülle) gegebenenfalls wiederum thermisch isoliert. Im Fall eines axial durchströmten im wesentlichen zylindrischen Schachtofens ist es für das erfindungsgemäße Verfahren im Fall einer adiabaten Betriebsweise vorteilhaft, wenn die Ausdehnung A des Reaktionsraums senkrecht zur zylindrischen Achse wenigstens das 5-fache, vorzugsweise wenigstens das 10-fache und besonders bevorzugt wenigstens das 15-fache der Schütthöhe S des wenigstens einen Katalysatorbettes in axialer Richtung beträgt. In der Regel wird das vorgenannte Verhältnis von A:S jedoch ≤ 200, üblicherweise ≤ 150 und meist ≤ 100 betragen.

Eine der erfindungsgemäßen Regenerierung vorausgehende, die Deaktivierung des erfindungsgemäß zu regenerierenden Katalysatorbetts bedingende, heterogen katalysierte Oxidehydrierung lässt sich prinzipiell so durchführen, wie zum Beispiel in den Schriften US-A 4,788,371, CN-A 1073893, Catalysis Letters 23 (1994), 103-106, W. Zhang, Gaodeng Xuexiao Huaxue Xuebao, 14 (1993) 566, Z. Huang, Shiyou Huagong, 21 (1992) 592, WO 97/36849, DE-A 197 53 817, US-A 3,862,256, US-A 3,887,631, DE-A 195 30 454, US-A 4,341,664, J. of Catalysis 167, 560-569 (1997), J. of Catalysis 167, 550-559 (1997), Topics in Catalysis 3 (1996) 265-275, US 5,086,032, Catalysis Letters 10 (1991), 181-192, Ind. Eng. Chem. Res. 1996, 35, 14-18, US 4,255,284, Applied Catalysis A: General, 100 (1993), 111-130, J. of Catalysis 148, 56-67 (1994), V. Cortés Corberán und S. Vic Bellón (Ed.), New Developments in Selective Oxidation II, 1994, Elsevier Science B.V., S. 305-313, 3rd World Congress on Oxidation Catalysis, R.K. Grasselli, S.T. Oyama, A.M. Gaffney and J.E. Lyons (Ed.), 1997, Elsevier Science B.V., S. 375ff oder in DE-A 198 37 520, DE-A 198 37 517, DE-A 198 37 519 und DE-A 198 37 518 am Beispiel der heterogen katalysierten partiellen Oxidehydrierung von Propan beschrieben. Dabei kann als Sauerstoffquelle z. B. Luft eingesetzt werden. Häufig weist die verwendete Sauerstoffquelle neben Inertgas hier jedoch zu mindestens 90 mol-% molekularen Sauerstoff, und vielfach zu wenigstens 95 mol-% molekularen Sauerstoff auf. Grundsätzlich kommt als Sauerstoffquelle auch reiner molekularer Sauerstoff in Betracht.

Die für die heterogen katalysierte Oxidehydrierung geeigneten Katalysatoren unterliegen, wie bereits gesagt, keinen besonderen Beschränkungen. Es eignen sich alle dem Fachmann auf diesem Gebiet bekannten Oxidehydrierungskatalysatoren, die in der Lage sind, z. B. Propan zu Propylen zu oxidieren. Insbesondere können alle in den zuvor genannten Schriften genannten Oxidehydrierungskatalysatoren eingesetzt werden. Geeignete Katalysatoren sind beispielsweise Oxidehydrierungskatalysatoren, die wenigstens V und Mg, oder wenigstens V und Sb, oder wenigstens V, Sb und Mg enthalten, aber auch Oxidehydrierungskatalysatoren, die MoVNb-Oxide oder Vanadylpyrophosphat, jeweils gegebenenfalls mit Promotor, umfassen. Ein Beispiel für einen günstigen Oxidehydrierkatalysator ist ein Katalysator, der ein Mischmetalloxid I mit Mo, V, Te, O und X als wesentliche Bestandteile enthält, wobei X mindestens ein Element ist, ausgewählt aus Niob, Tantal, Wolfram, Titan, Aluminium, Zirkonium, Chrom, Mangan, Gallium, Eisen, Ruthenium, Kobalt, Rhodium, Nickel, Palladium, Platin, Antimon, Bismut, Bor, Indium, Silizium, Lanthan, Natrium, Lithium, Kalium, Magnesium, Silber, Gold und Cer (vgl. dazu auch EP-A 938463 und EP-A 167109). Weitere besonders geeignete Oxidehydrierungskatalysatoren sind die Multimetalloxidmassen beziehungsweise -katalysatoren A (in dieser Schrift Multimetalloxidmassen II genannt) der DE-A-197 53 817 und die Katalysatoren der DE-A 19838312, wobei die in der erstgenannten Schrift als bevorzugt genannten Multimetalloxidmassen beziehungsweise-katalysatoren A ganz besonders günstig sind. Damit kommen für eine heterogen katalysierte Oxidehydrierung eines zu dehydrierenden Kohlenwasserstoffs als Aktivmassen u.a. Multimetalloxidmassen der allgemeinen Formel III

$$M^1_a MO_{1-b} M^2_b O_x \qquad \text{(III)},$$

mit

$M^1$ = Co, Ni, Mg, Zn, Mn und/oder Cu,
$M^2$ = W, V, Te, Nb, P, Cr, Fe, Sb, Ce, Sn und/oder La,
a = 0,5-1,5,
b = 0-0,5,

sowie

x = eine Zahl, die durch die Wertigkeit und Häufigkeit der von Sauerstoff verschiedenen Elemente in (III) bestimmt wird, in Betracht. Ihre Herstellung und Formgebung kann wie in der DE-A 102 45 585 beschrieben erfolgen.

Für eine heterogen katalysierte Oxidehydrierung von z. B. Propan liegt die Reaktionstemperatur bei Verwendung frischer Katalysatoren vorzugsweise im Bereich von 200 bis 600°C, insbesondere im Bereich von 250 bis 500°C, stärker bevorzugt im Bereich von 350 bis 440°C. Der Arbeitsdruck liegt vorzugsweise im Bereich von 0,5 bis 10 bar, insbesondere von 1 bis 10 bar, stärker bevorzugt von 1 bis 5 bar. Arbeitsdrucke oberhalb von 1 bar, zum Beispiel von 1,5 bis 10 bar, haben sich als besonders vorteilhaft erwiesen. In der Regel erfolgt auch die heterogen katalysierte Oxidehydrierung des z.B. Propans (Propan wird hier stets stellvertretend für alle anderen zu dehydrierenden Kohlenwasserstoffe benannt) an einem Katalysatorfestbett. Letzteres wird zweckmäßigerweise in den Rohren (die Rohrwand bildet zusammen mit den beiden Rohröffnungen die den Reaktionsraum berührende Einhüllende; das Rohrinnere ist der Reaktionsraum;) eines z. B. salzbadgekühlten Rohrbündelreaktors aufgeschüttet (in der Regel auf einem gasdurchlässigen Gitterrost befindlich), wie sie zum Beispiel in der EP-A 700 893 und in der EP-A 700 714 sowie der in diesen Schriften zitierten Literatur beschrieben sind. Der Ausgangsgasstrom wird dem Rohreingang zugeführt. Die mittlere Verweilzeit des Reaktionsgases in der Katalysatorschüttung liegt zweckmäßigerweise bei 0,5 bis 20 Sekunden. Das Verhältnis von z.B. Propan zu Sauerstoff variiert mit dem gewünschten Umsatz und der Selektivität des Katalysators. Zweckmäßigerweise liegt es im Bereich von 0,5:1 bis 40:1, insbesondere von 1:1 bis 15:1, stärker bevorzugt von 2:1 bis 6:1 bzw. bis 5:1. In der Regel nimmt die Propylenselektivität mit steigendem Propanumsatz ab. Vorzugsweise wird deshalb die Propan zu Propylen-Reaktion so durchgeführt, dass relativ niedrige Umsätze mit Propan bei hohen Selektivitäten zu Propylen erreicht werden. Besonders bevorzugt liegt der Umsatz an Propan im Bereich von 5 bis 40 mol-%, häufig im Bereich von 10 bis 30 mol-%. Hierbei bedeutet der Begriff "Propanumsatz" den Anteil an zugeführtem Propan, der beim einfachen Durchgang des Reaktionsgases durch das Rohr umgesetzt wird. In der Regel beträgt die Selektivität der Propylenbildung 50 bis 98 bzw. bis 99 mol-%, stärker bevorzugt 80 bis 98 bzw. 99 mol-%, wobei der Begriff "Selektivität" die Mole an Propylen bezeichnet, die pro Mol umgesetztem Propan erzeugt werden, ausgedrückt als molarer Prozentsatz. Im Reaktionsrohr durchläuft die Reaktionstemperatur in der Regel ein Maximum.

In der Regel enthält der bei einer heterogen katalysierten Propanoxidehydrierung eingesetzte Ausgangsgasstrom 5 bis 95 mol-% Propan (bezogen auf 100 mol-% Ausgangsgas). Neben Propan (bzw. einem anderen zu oxidehydrierenden Kohlenwasserstoff) und Sauerstoff kann das Ausgangsgas für die heterogen katalysierte Oxidehydrierung auch weitere, insbesondere inerte, Bestandteile wie Kohlendioxid, Kohlenmonoxid, Stickstoff, Edelgase, andere Kohlenwasserstoffe, z. B. im Roh-Propan (als Propanquelle wird für das erfindungsgemäße Verfahren normalerweise Roh-Propan verwendet, wie es z. B. in der DE-A 102 45 585 bzw. in der DE-A 10 2005 022 798 empfohlen wird) enthaltene Nebenbestandteile, und/oder Propylen umfassen. Die heterogen katalysierte Oxidehydrierung kann auch in Anwesenheit von Verdünnungsmitteln, wie zum Beispiel Wasserdampf, durchgeführt werden.

Jede beliebige Reaktorsequenz kann zur Durchführung der heterogen katalysierten Oxidehydrierung von z. B. Propan eingesetzt werden, die dem Fachmann bekannt ist. Zum Beispiel kann die heterogen katalysierte Oxidehydrierung in einem einzigen Reaktor oder in einer Kaskade aus zwei oder mehreren Reaktoren, zwischen denen gegebenenfalls Sauerstoff zugeführt wird, durchgeführt werden.

Als mögliche Bestandteile kann das Produktgas einer wie beschrieben durchzuführenden heterogen katalysierten Propanoxidehydrierung zum Beispiel folgende Bestandteile enthalten: Propylen (als Zielprodukt, d.h., als dehydrierter Kohlenwasserstoff), Propan (als nicht umgesetzter zu dehydrierender Kohlenwasserstoff), Kohlendioxid, Kohlenmonoxid, Wasser, Stickstoff, Sauerstoff, Ethan, Ethen, Methan, Acrolein, Acrylsäure, Methacrolein, Methacrylsäure, Furfurale, Ethylenoxid, Butan (z. B. n-Butan oder iso-Butan), Essigsäure, Formaldehyd, Ameisensäure, Propylenoxid und Butene (z. B. Buten-1). Dabei bilden insbesondere Ethan, Ethen und Methan mögliche thermische Zersetzungsprodukte von Propan. In typischer Weise enthält ein bei einer erfindungsgemäßen heterogen katalysierten Propanoxidehydrierung erhaltenes Produktgas: 5 bis 10 mol-% Propylen, 0,1 bis 2 mol-% Kohlenmonoxid, 1 bis 3 mol-% Kohlendioxid, 4 bis 10 mol-% Wasser, 0 bis 10 bzw. bis 1 mol-% Stickstoff, 0,01 (bzw. 0) bis 0,5 mol-% Acrolein, 0,01 (bzw. 0) bis 1 mol-% Acrylsäure, 0,05 (bzw. 0) bis 2 mol-% Essigsäure, 0,01 (bzw. 0) bis 0,05 mol-% Formaldehyd, 1 bis 5 mol-% Sauerstoff, 0,1 bis 10 mol-% weitere oben genannte Bestandteile, sowie als Rest im wesentlichen Propan, jeweils bezogen auf 100 % Produktgas.

Heterogen katalysierte Oxidehydrierungen von von Propan verschiedenen zu dehydrierenden Kohlenwasserstoffen können erfindungsgemäß in entsprechender Weise durchgeführt werden, wie vorstehend für die Oxidehydrierung von Propan beschrieben. Als solche zu oxidehydrierenden Kohlenwasserstoffe kommen insbesondere Butan (zu Buten (vor allem iso-Butan zu iso-Buten) und/oder Butadien) sowie Butene (zu Butadien) in Betracht.

Häufig wird sich an die oxidative oder nicht oxidative heterogen katalysierte Dehydrierung des zu dehydrierenden Kohlenwasserstoffs ein Verfahren der heterogen katalysierten Partialoxidation von in selbiger erzeugtem dehydriertem Kohlenwasserstoff (z.B. Propylen zu Acrolein und/oder Acrylsäure), vorzugsweise in Begleitung von in der Dehydrierung nicht umgesetztem zu dehydrierendem Kohlenwasserstoff (z.B. Propan) als Inertgas bezüglich einer solchen Partialoxidation anschließen.

Dabei wird man den aus der Dehydrierung (kontinuierlich) entnommenen Produktgasstrom A als solchen oder nach Abtrennung wenigstens einer Teilmenge seiner vom dehydrierten Kohlenwasserstoff (z. B. Propylen) und vom (nicht umgesetzten) zu dehydrierenden Kohlenwasserstoff (z. B. Propan) verschiedenen Bestandteile (z. B. $H_2$, $H_2O$, $N_2$, etc.) zur Beschickung wenigstens eines Oxidationsreaktors verwenden, und den im Beschickungsgasgemisch enthaltenen dehydrierten Kohlenwasserstoff (z. B. Propylen) einer selektiven heterogen katalysierten partiellen Gasphasenoxidation mit molekularem Sauerstoff zu einem das (Partialoxidationsprodukt) Zielprodukt (z. B. Acrolein, oder Acrylsäure oder deren Gemisch), sowie in der Regel nicht umgesetzten zu dehydrierenden Kohlenwasserstoff (z. B. Propan), überschüssigen molekularen Sauerstoff und gegebenenfalls nicht umgesetzten dehydrierten Kohlenwasserstoff (z. B. Propylen) enthaltenden Produktgasgemisch B unterwerfen.

In einer nachfolgenden Trennzone B wird man im Produktgasgemisch B enthaltenes Zielprodukt (z. B. Acrolein, oder Acrylsäure, oder deren Gemisch) abtrennen, und von dem dabei verbleibenden, nicht umgesetzten zu dehydrierenden Kohlenwasserstoff (z. B. Propan), molekularen Sauerstoff sowie gegebenenfalls nicht umgesetzten dehydrierten Kohlenwasserstoff (z. B. Propylen) enthaltenden Restgas wenigstens eine nicht umgesetzten zu dehydrierenden Kohlenwasserstoff (z. B. Propan) und gegebenenfalls nicht umgesetzten molekularen Sauerstoff sowie gegebenenfalls nicht umgesetzten dehydrierten Kohlenwasserstoff (z. B. Propylen) enthaltende Teilmenge als Partialoxidationskreisgas in die Dehydrierung rückführen (z. B. als Bestandteil des Ausgangsgasstroms).

Handelt es sich bei der Dehydrierung z. B. um eine oxidative konventionelle heterogen katalysierte partielle Dehydrierung von Propan zu Propylen, und bei der sich anschließenden Partialoxidation um diejenige von Propylen zu Acrolein, oder zu Acrylsäure, oder zu deren Gemisch, kann der der oxidativen Dehydrierung zugeführte Ausgangsgasstrom z. B. als wesentliche Gehalte enthalten:

| | |
|---|---|
| Propylen | $\geq 0$ bis 20 bzw. bis 10, häufig 0 bis 6 Vol.-%, |
| Acrolein | $\geq 0$ bis 1, vielfach 0 bis 0,5, häufig 0 bis 0,25 Vol.-%, |
| Acrylsäure | $\geq 0$ bis 0,25 (oder bis 0,4), vielfach 0 bis 0,05, häufig 0 bis 0,03 Vol.-%, |
| $CO_x$ | $\geq 0$ bis 20 bzw. bis 5, vielfach 0 bis 3, häufig 0 bis 2 Vol.-%, |
| Propan | 5 bis 50, vorzugsweise 20 bis 40 Vol.-%, |
| Stickstoff | 20 bzw. 30 bis 80, vorzugsweise 50 bis 70 Vol.-%, |
| Sauerstoff | $\geq 0$ bis 5, vorzugsweise 1,0 bis 2,0 Vol.-%, |
| $H_2O$ | $\geq 0$ bis 20, vorzugsweise 5,0 bis 10,0 Vol.-%, und |
| $H_2$ | $\geq 0$, häufig $\geq 0,01$, oft $\geq 0,05$ bis 10, vorzugsweise 1 bis 5 Vol.-%. |

In geringen Menge (etwa vergleichbar den möglichen Acrylsäuregehalten) kann auch Essigsäure enthalten sein.

Die aus der Partialoxidation des dehydrierten Kohlenwasserstoffs herrührenden Bestandteile im der Dehydrierung dann zugeführten Ausgangsgasstrom vermögen ihrerseits zur Deaktivierung des in der Dehydrierung verwendeten Katalysators (Katalysatorbetts) beizutragen. Das erfindungsgemäße Regenerierungsverfahren erweist sich jedoch auch in diesen Fällen als wirksam.

Beispielsweise findet man solche mehrstufigen Verfahren beschrieben in den Schriften DE-A 10 2006 029 790, DE-A 10 2006 035 718, DE-A 10 2005 022 798, Deutsches Aktenzeichen 10 2006 024 901.1, DE-A 102 46 119, DE-A 102 45 585, DE-A 10 2005 049 699, DE-A 10 2004 032 129, DE-A 10 2005 013 039, DE-A 10 2005 010 111, DE-A 10 2005 009 891, DE-A 102 11 275, EP-A 117 146, US 3,161,670, DE-A 33 13 573, WO 01/96270, DE-A 103 16 039, DE-A 102 19 686, DE-A 10 2005 009 885, DE-A 10 2005 052 923, DE-A 10 2005 057 197, WO 03/076370, DE-A 102 45 585, DE-A 22 13 573, US 3,161,670 und dem in diesen Schriften zitierten Stand der Technik.

Die Vorteilhaftigkeit des erfindungsgemäßen Regenerierverfahrens beruht vermutlich darauf, dass die im Verlauf der Deaktivierung auf und im Katalysator(fest)bett abgeschiedenen, die Deaktivierung mit bedingenden, unterschiedlichen an Kohlenstoff reichen Spezies quasi sukzessive (d.h., nacheinander, aufeinanderfolgend) abgebrannt werden können. Das erfindungsgemäße Verfahren zeichnet sich insbesondere auch dadurch aus, dass das Deaktivierungsverhalten des erfindungsgemäß regenerierten Katalysatorbetts sich vom Deaktivierungsverhalten des frisch beschickten Katalysatorbetts im wesentlichen nicht unterscheidet. Betriebsdauern von 30 bis 100 h zwischen zwei Regenerierungen sind typisch.

Ausführungsbeispiel und Vergleichsbeispiele

I. Ausführungsbeispiel

a) Beschreibung des Dehydrierreaktors

Als Dehydrierreaktor wurde ein Stahlrohr (Edelstahl der DIN Werkstoffnummer 1.4841) der Länge 2 070 mm, der Wanddicke 1 mm und des Innendurchmessers 36,4 mm verwendet. Der Rohrreaktor wurde vom Reaktionsgasgemischstrom von oben nach unten durchströmt. Ins untere Ende des Rohrreaktors ragte ein 115 mm hoher Kontaktstuhl aus demselben Edelstahl, der das Katalysatorgesamtfestbett (bestehend aus drei Katalysatorteilfestbetten von ca. identischem Schüttgewicht) wie folgt von oben nach unten strukturiert trug:

| | |
|---|---|
| 790 mm | Schüttlänge aus (inerten) Steatitkugeln (Durchmesser 4 - 5 mm) des Steatits C-220 der Fa. CeramTec (interne Aufheizschüttung); |
| 195 mm | Schüttlänge Dehydrierkatalysator (Pt/Sn-Legierung, die mit den Elementen Cs, K und La in oxidischer Form promoviert war und die auf die äußere und innere Oberfläche von $ZrO_2 \cdot SiO_2$ Mischoxidträgerstränglingen (mittlere Länge (gaußverteilt im Bereich von 3 mm bis 12 mm mit Maximum bei ca. 6 mm) : 6 mm, Durchmesser: 2 mm) in der Elementstöchiometrie (Massenverhältnis einschließlich Träger) $Pt_{0,3}Sn_{0,6}La_{3,0}Cs_{0,5}K_{0,2}(ZrO_2)_{88,3}(SiO_2)_{7,1}$ aufgebracht war (Katalysatorvorläuferherstellung und Aktivierung zum aktiven Katalysator wie in Beispiel 4 der DE-A 102 19 879); |
| 145 mm | Schüttlänge aus Steatitkugeln (Durchmesser 4 - 5 mm) des Steatits C-220 der Fa. CeramTec; |
| 190 mm | Schüttlänge des vorgenannten Dehydrierkatalysators; |
| 145 mm | Schüttlänge aus Steatitkugeln (Durchmesser 4 - 5 mm) des Steatits C-220 der Fa. CeramTec; |
| 195 mm | Schüttlänge des vorgenannten Dehydrierkatalysators; |
| 20 mm | Schüttlänge aus Steatitkugeln (Durchmesser 1,5 - 2,5 mm) des Steatits C-220 der Fa. CeramTec; und |
| 210 mm | Schüttlänge aus Steatitkugeln (Durchmesser 4 - 5 mm) des Steatits C-220 der Fa. CeramTec. |

Oberhalb der internen Aufheizschüttung war das Stahlrohr leer.

Der Rohrreaktor war außen im Sinne einer Vorheizstrecke auf der Länge der obersten 400 mm der Aufheizschüttung von oben nach unten (zum Kontaktstuhl hin) in zwei, eine Gleichverteilung der zugeführten Wärmemenge gewährleistende, Halbschalen aus Kupfer (Schalendicke = 25 mm) eingelegt, die mittels eines sie im vollen Umfang umgebenden Heizbandes elektrisch beheizt wurden. Um das Heizband befand sich eine Wicklung aus thermischem Isoliermaterial.

Von unten nach oben (kurz unterhalb der Kontaktstuhlauflagefläche beginnend) war der Rohrreaktor auf einer Länge von 1 530 mm in zwei Paare von thermisch isolierend wirkenden Halbschalen (Dicke einer Halbschale = 25 mm) aus MPS-Super G der Fa. Microtherm in DE, die um 90° gegeneinander versetzt übereinander angebracht waren, eingebracht. Die isolierend wirkenden Halbschalen waren ihrerseits von einer zylindrischen Einhüllenden aus Edelstahl (Außendurchmesser = 168 mm, Innendurchmesser = 154 mm) umgeben, um die herum zum Zweck der Begleitheizung ein Strahlungsofen (Länge = 1 600 mm) angeordnet war. Auf diese Weise konnte auf der adiabaten Strecke der Wärmefluss aus der Umgebung in das Reaktionsrohr hinein und aus dem Reaktionsrohr heraus in die Umgebung minimiert werden.

In das Reaktionsrohr war mittig (zentral) zusätzlich eine 2 500 mm lange Thermohülse eingeführt (Außendurchmesser = 6 mm; Innendurchmesser = 4 mm), in die ein Mehrfach-Thermoelement (vom unteren Reaktorende nach oben alle 8 cm insgesamt 14 Messstellen, Dicke 3,2 mm) eingeführt war.

Zusätzlich waren zwei Lanzenpaare in den Rohrreaktor eingeführt. Das eine Paar war von unten und das andere Paar von oben in den Rohrreaktor eingeführt. Die Lanzen eines Paares wurden jeweils aneinanderliegend zwischen Thermohülse und Reaktorinnenwand so geführt, dass ihre Positionierung über den Rohrquerschnitt zwischen Thermohülse und Reaktorinnenwand mittig war. In eine Rohrquerschnittsebene projiziert standen sich die beiden Paare auf einem Rohrdurchmesser gegenüber. Der Außendurchmesser einer aus Edelstahl der DIN Werkstoffnummer 1.4841 gefertigten Lanze betrug 3,17 mm und ihr Innendurchmesser lag bei 2,17 mm. Die Länge der Lanzen eines Paares war unterschiedlich. Durch jeweils eine der beiden Lanzen eines Lanzenpaares wurde Luft zudosiert und durch die jeweils andere Lanze eines Lanzenpaares wurde Reaktionsgasgemisch zu Analysezwecken entnommen. Die in Strömungsrichtung erste Analysenlanze (LI) war mit ihrer Öffnung 20 mm hinter dem in Strömungsrichtung ersten Katalysatorteilfestbett platziert. Die zugehörige Zudosierungslanze (ZI) war mit ihrer Öffnung 100 mm vor dem in Strömungsrichtung zweiten Katalysatorteilfestbett platziert. Die in Strömungsrichtung zweite Analysenlanze (LII) war mit ihrer Öffnung 20 mm hinter dem in Strömungsrichtung zweiten Katalysatorteilfestbett platziert. Die zugehörige Zudosierungslanze (ZII) war mit ihrer Öffnung 100 mm vor dem in Strömungsrichtung dritten Katalysatorteilfestbett platziert.

Vor dem Rohrreaktor war ein mit Steatitkugeln (aus Steatit C-220 der Fa. CeramTec, Durchmesser 4 - 5 mm) gefülltes Stahlrohr der Länge 1 300 mm als Erhitzer geschaltet. In ihm wurde der Reaktionsgasgemischstrom auf seine Eintrittstemperatur in den Rohrreaktor vorgeheizt und gleichzeitig ideal durchmischt. Zu diesem Zweck wurde das Erhitzerrohr (Edelstahl der DIN Werkstoffnummer 1.4841, Wanddicke 3,6 mm, Innendurchmesser 53,1 mm) auf einer rohrmittigen Länge von 1 200 mm mittels um sie angelegter Heizmanschetten der Fa. Horst, DE-Heidelberg elektrisch beheizt. Die Verbindung zwischen Erhitzer und Rohrreaktor wurde durch ein thermisch beheiztes und mit üblichen Wärmedämmmaterialien thermisch isoliertes Edelstahlrohr (Edelstahl der DIN Werkstoffnummer 1.4841, Außendurchmesser 14 mm, Innendurchmesser 10 mm, Länge 300 mm) bewerkstelligt.

b) Beginn des Langzeitbetriebs (Betriebszeitpunkt $t_0$) der heterogen katalysierten partiellen Dehydrierung von Propan

Das Katalysatorgesamtbett war mit frischem Dehydrierkatalysator beschickt. Der dem Katalysatorgesamtfestbett zugeführte Reaktionsgasgemischstrom war ein Gemisch aus Roh-Propan, Wasserdampf und Partialoxidationskreisgas einer heterogen katalysierten zweistufigen Partialoxidation des in der Dehydrierung erzeugten Propylens zu Acrylsäure. Aus dem Produktgasgemisch der Dehydrierung wurden die von Propan und Propylen verschiedenen Bestandteile wie im Vergleichsbeispiel 1 der deutschen Anmeldung Nr. 10 2005 013 039 beschrieben absorptiv/desorptiv (Strippen mit Luft) abgetrennt. Die Partialoxidation des Propylens zu Acrylsäure erfolgte ebenfalls wie in der deutschen Anmeldung Nr. 10 2005 013 039 beschrieben. Das gleiche gilt für die Bildung des Partialoxidationskreisgases.

Der Reaktionsgasgemischstrom wurde wie in der deutschen Anmeldung Nr. 10 2005 013 039 beschrieben mit einer Austrittstemperatur von etwa 200 °C in einem Verdampfer erzeugt und von diesem ausgehend dem Erhitzer zugeführt (die Anbindung des Verdampfers an den Erhitzer war wie diejenige des Erhitzers an den Rohrreaktor gestaltet).

Die Beheizung des Erhitzers war so geregelt, dass der vom Verdampfer in den Erhitzer geleitete Reaktionsgasgemischstrom diesen mit einer Temperatur von etwa 400 °C verließ. Dann wurde der Reaktionsgasgemischstrom in den Rohrreaktor geführt und in der Vorheizstrecke desselben weiter erhitzt und schließlich durch den Rohrreaktor geführt. Über die Zudosierungslanzen ZI und ZII wurden jeweils etwa 30 Nl/h Luft zudosiert, die am Eingang in die jeweilige Lanze eine Temperatur von 25 °C aufwies. Nach einer Betriebszeit von to = 1 h erreichte der Verfahrensbetrieb im wesentlichen erstmals seinen (quasi) stationären Betriebszustand.

Die Stoffgehalte des dem Rohrreaktor zugeführten Reaktionsgasgemischstroms (alle Gaszusammensetzungsangaben in dieser Schrift basieren stets auf gaschromatographischer Analyse) von 2 807 Nl/h sowie die Stoffgehalte des Reaktionsgasgemischstroms beim Verlassen des in Strömungsrichtung ersten, zweiten sowie dritten Katalysatorteilfestbetts waren dabei wie in der Tabelle 1 angegeben.

Tabelle 1

| | Reaktoreingang | nach 1. Teilbett | nach 2. Teilbett | nach 3. Teilbett |
|---|---|---|---|---|
| Vol.ppm Acrolein | 235 | <2 | <2 | <2 |
| Vol.ppm Acrylsäure | 344 | <2 | <2 | <2 |
| Vol.ppm Essigsäure | 170 | <2 | <2 | <2 |
| Vol.-% Wasserstoff | 0,05 | 2,42 | 3,15 | 4,37 |
| Vol.-% Sauerstoff | 2,93 | <2 | <2 | <2 |
| Vol.-% Stickstoff | 53 | 51 | 51 | 51 |
| Vol.-% Kohlenmonoxid | 0,36 | 0,15 | 0,16 | 0,18 |
| Vol.-% Kohlendioxid | 1,29 | 2,20 | 2,53 | 2,48 |
| Vol.ppm Methan | 39 | 175 | 342 | 386 |
| Vol.ppm Ethan | 305 | 313 | 357 | 447 |
| Vol.ppm Ethen | 328 | 397 | 565 | 599 |
| Vol-ppm Acetylen | 2 | 14 | 25 | 26 |
| Vol.-% Propan | 35,4 | 32,5 | 29,6 | 27,8 |
| Vol.ppm Cyclopropan | 200 | 85 | 35 | 19 |

(fortgesetzt)

| | Reaktoreingang | nach 1. Teilbett | nach 2. Teilbett | nach 3. Teilbett |
|---|---|---|---|---|
| Vol.-% Propen | 0,34 | 4,92 | 6,90 | 7,33 |
| Vol.ppm Propadien | <2 | <2 | <2 | <2 |
| Vol.ppm Propin | <2 | 28 | 50 | 52 |
| Vol.ppm iso-Butan | 71 | 63 | 57 | 51 |
| Vol.ppm n-Butan | 3 | 3 | 3 | 3 |
| Vol.ppm trans-Buten-2 | 2 | 2 | 2 | 3 |
| Vol.ppm Buten-1 | < 2 | < 2 | 2 | 3 |
| Vol.ppm iso-Buten | 5 | 7 | 12 | 17 |
| Vol.ppm cis-Buten-2 | < 2 | < 2 | 2 | 2 |
| Vol.pmm Butadien-1.3 | 7 | 2 | 2 | 2 |
| Vol.ppm sonstige $C_2$-$C_4$-Kohlenwasserstoffe | < 2 | < 2 | < 2 | < 2 |
| Vol.ppm Benzol | 83 | 69 | 69 | 78 |
| Vol.ppm Restsumme $C_5$-Kohlenwasserstoffe | 34 | 9 | 10 | 17 |
| Vol.ppm Reststumme $C_6$-Kohlenwasserstoffe | 8 | 7 | 7 | 5 |
| Restmenge bis zu 100 Vol.-% = jeweils Wasser | | | | |

Beim Durchgang des Reaktionsgasgemischstroms durch das Katalysatorgesamtbett wurden insgesamt G = (A + B + C) mol-% der in diesem enthaltenen molaren Ausgangsmenge KW an zu dehydrierendem Kohlenwasserstoff zu dehydriertem Kohlenwasserstoff dehydriert. A ist dabei der Beitrag des in Strömungsrichtung ersten Katalysatorteilfestbetts, B ist der Beitrag des in Strömungsrichtung zweiten Katalysatorteilfestbetts und C ist der Beitrag des in Strömungsrichtung dritten Katalysatorteilfestbetts.

Zum Betriebszeitpunkt to lauteten die numerischen Werte für G, A, B und C wie folgt:

A = 12 mol-%,
B = 5,4 mol-%,
C = 1,8 mol-%, und
G = 19,2 mol-%,

jeweils bezogen auf KW.

Die Temperatur $T_1$ des Reaktionsgasgemischstroms in Strömungsrichtung unmittelbar hinter der internen Aufheizschüttung im Rohrreaktor betrug zum Betriebszeitpunkt $t_0$ = 408°C. Die zum Betriebszeitpunkt to über ZI und ZII zudosierten Luftmengenströme MI und MII waren jeweils ca. 33 Nl/h.

c) Durchführung des Langzeitbetriebs der heterogen katalysierten partiellen Dehydrierung von Propan

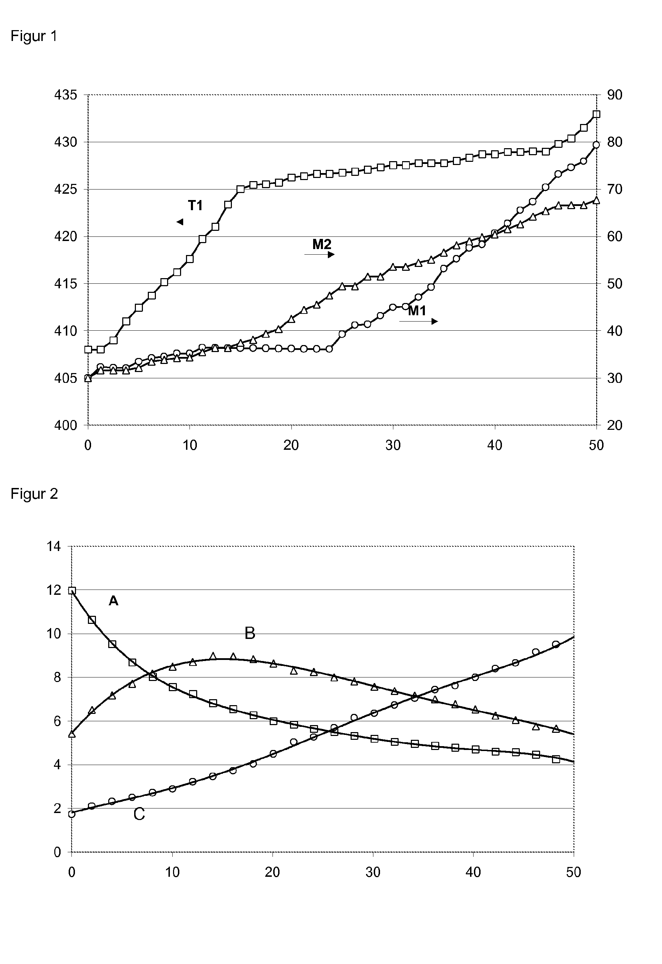

Um einer mit zunehmender Betriebsdauer einhergehenden Deaktivierung des Katalysatorgesamtfestbetts entgegenzuwirken, wurden sowohl $T_1$ als auch MI (= M1 in Figur 1) und MII (= M2 in Figur 1) mit zunehmender Betriebsdauer wie in Figur 1 (anhand der erhobenen Messpunkte) gezeigt sukzessive angehoben (linke Ordinate = $T_1$ in °C; rechte Ordinate = Luftmengenstrom in Nl/h; der Nullpunkt wurde in $t_0$ gelegt; die Abszisse zeigt t in h). Auf diese Art und Weise gelang es über das Betriebszeitpunktintervall 0 h = to < t ≤ 50 h den Wert für G im Intervall G = 19,2 ± 0,2 mol-% stabil zu halten. Den dabei resultierenden zeitlichen Verlauf (die Abszisse zeigt t in h) der erhobenen Umsatzanteile A, B, C (mol-% von KW als Ordinate) im Betriebszeitpunktintervall zeigt Figur 2.

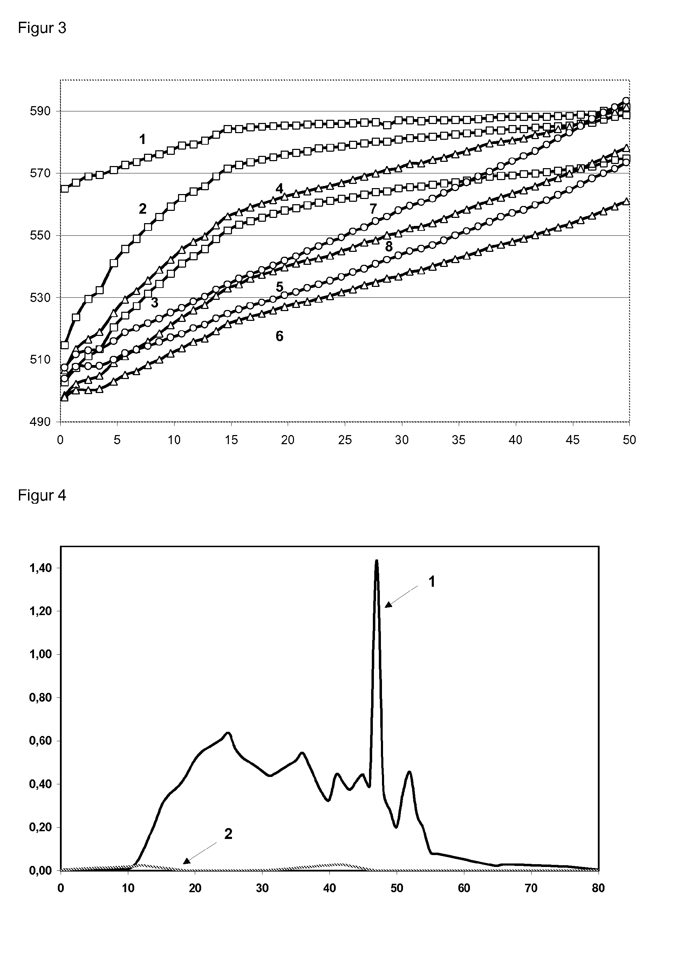

Den dabei resultierenden zeitlichen Verlauf der Temperatur des Reaktionsgasgemischstroms kurz hinter dem Eingang (die maximale Temperatur im Teilbett, Bezugsziffer 1), in der Mitte (Bezugsziffer 2) und am Ausgang (Bezugsziffer 3) des in Strömungsrichtung ersten Katalysatorteilfestbetts zeigt (anhand der erhobenen Messwerte) die Figur 3 (die Abszisse zeigt wieder t in h, der Nullpunkt wurde in $t_0$ gelegt, und die Ordinate zeigt T in °C) ebenso, wie den zeitlichen Verlauf der Temperatur des Reaktionsgasgemischstroms kurz hinter dem Eingang (die maximale Temperatur im Teilbett, Bezugsziffer 4), in der Mitte (Bezugsziffer 5) und am Ausgang (Bezugsziffer 6) des in Strömungsrichtung zweiten Katalysatorteilfestbetts bzw. des in Strömungsrichtung dritten Katalysatorteilfestbetts (Bezugsziffer 7 = die maximale Temperatur im dritten Katalysatorteilfestbett kurz hinter dem Eingang ins selbige, Bezugsziffer 8 = kurz vor dem Ausgang des dritten Katalysatorteilfestbetts).

d) Durchführung einer erfindungsgemäßen Regenerierung

Nach einer Betriebszeit von t = to + 50 h wurden die Luftmengenströme MI und MII unterbrochen und der dem Rohrreaktor zugeführte Reaktionsgasgemischstrom durch einen Stickstoffstrom zum Spülen des Katalysatorbetts ersetzt. Die Reinheit des Stickstoffstroms war ≥ 99,9 Vol.-%.

Der Stickstoffstrom betrug 3930 Nl/h. Die Beheizung mittels Erhitzer und Vorheizstrecke war so geregelt, dass die Temperatur des Stickstoffstroms in Strömungsrichtung unmittelbar hinter der internen Aufheizschüttung im Rohrreaktor 450°C betrug. Der Verlassdruck des Stickstoffstroms am Ausgang des Rohrreaktors lag 2 bar über Atmosphärendruck. Die Verlassdruckregelung erfolgte mittels eines Druckhalteregelventils.

Die vorgenannten Bedingungen wurden über einen Zeitraum von 10 h beibehalten. Anschließend wurde der Stickstoffstrom durch 4102 Nl/h eines Gemischs aus 3930 Nl/h Stickstoffstrom und 172 Nl/h Luft ersetzt. Die Temperatur dieses Regeneriergasstroms wurde in Strömungsrichtung unmittelbar hinter der internen Aufheizschüttung ebenfalls auf 450°C eingestellt. Der Verlassdruck des Regeneriergasstroms am Ausgang des Rohrreaktors lag 3,5 bar über Atmosphärendruck.

Die vorgenannten Bedingungen wurden über einen Zeitraum von 24 min beibehalten. Dann wurde unter Beibehalt der übrigen Regenerierbedingungen die Zusammensetzung und der Betrag des Regeneriergasstroms jeweils für eine Zeitdauer t in der nachfolgend aufgelisteten Abfolge verändert.:

| Zeitdauer t (min) | Regeneriergasstrom (Nl/h) | Anteil Luftstrom (Nl/h) | Anteil Stickstoffstrom (Nl/h) |
|---|---|---|---|
| 5 | 4145 | 215 | 3930 |
| 9 | 4253 | 323 | 3930 |
| 7 | 4360 | 430 | 3930 |
| 2 | 4468 | 538 | 3930 |
| 4 | 4576 | 646 | 3930 |
| 4 | 4791 | 861 | 3930 |
| 5 | 5006 | 1076 | 3930 |
| 6 | 5545 | 1615 | 3930 |
| 4 | 4845 | 1615 | 3230 |
| 3 | 4307 | 1615 | 2692 |
| 2 | 3769 | 1615 | 2154 |
| 4 | 3230 | 1615 | 1615 |
| 2 | 2691 | 1615 | 1076 |
| 3 | 2153 | 1615 | 538 |
| 23 | 1615 | 1615 | 0 |

Anschließend wurde die Temperatur des Regeneriergasstroms in Strömungsrichtung unmittelbar hinter der internen Aufheizschüttung auf 550°C eingestellt und unter Beibehalt dieser Temperatur die Zusammensetzung und der Betrag des Regeneriergasstroms sowie der Verlassdruck P (bar über Atmosphärendruck) jeweils für eine Zeitdauer t in der nachfolgend aufgelisteten Abfolge verändert:

| Zeitdauer t (min) | Regeneriergasstrom (Nl/h) | Anteil Luftstrom (Nl/h) | Anteil Stickstoffstrom (Nl/h) | P (ü bar) |
|---|---|---|---|---|
| 156 | 1615 | 1615 | 0 | 3,5 |

(fortgesetzt)

| Zeitdauer t (min) | Regeneriergasstrom (NI/h) | Anteil Luftstrom (NI/h) | Anteil Stickstoffstrom (NI/h) | P (ü bar) |
|---|---|---|---|---|
| 1 | 0 | 0 | 0 | 0 |
| 9 | 108 | 108 | 0 | 0 |
| 114 | 1615 | 1615 | 0 | 3,5 |
| 10 | 1615 | 1615 | 0 | 0 |
| 170 | 1615 | 1615 | 0 | 3,5 |
| 5 | 1615 | 1615 | 0 | 0 |
| 180 | 1615 | 1615 | 0 | 3,5 |
| 10 | 1615 | 1615 | 0 | 0 |
| 170 | 1615 | 1615 | 0 | 3,5 |
| 10 | 1615 | 1615 | 0 | 0 |
| 170 | 1615 | 1615 | 0 | 3,5 |
| 10 | 1615 | 1615 | 0 | 0 |
| 170 | 1615 | 1615 | 0 | 3,5 |
| 10 | 1615 | 1615 | 0 | 0 |
| 170 | 1615 | 1615 | 0 | 3,5 |
| 10 | 1615 | 1615 | 0 | 0 |
| 165 | 1615 | 1615 | 0 | 3,5 |
| 10 | 1615 | 1615 | 0 | 0 |
| 175 | 1615 | 1615 | 0 | 3,5 |
| 10 | 1615 | 1615 | 0 | 0 |
| 160 | 1615 | 1615 | 0 | 3,5 |
| 10 | 1615 | 1615 | 0 | 0 |
| 170 | 1615 | 1615 | 0 | 3,5 |
| 10 | 1615 | 1615 | 0 | 0 |
| 170 | 1615 | 1615 | 0 | 3,5 |
| 10 | 1615 | 1615 | 0 | 0 |
| 170 | 1615 | 1615 | 0 | 3,5 |
| 10 | 1615 | 1615 | 0 | 0 |
| 180 | 1615 | 1615 | 0 | 3,5 |
| 10 | 1615 | 1615 | 0 | 0 |
| 1280 | 1615 | 1615 | 0 | 2 |
| 5 | 1615 | 1615 | 0 | 0 |

Während der letzten 5 Minuten wurde die Temperatur des Regeneriergasstroms in Strömungsrichtung unmittelbar hinter der internen Aufheizschüttung auf 450°C eingestellt.

Dann wurde als Reduziergas im Verdampfer ein Gemisch aus 10,8 g/h $H_2O$, 430 NI/h Stickstoff und 430 NI/h Wasserstoff (Reinheit > 99,9 Vol.-%) erzeugt. Die Temperatur des Reduziergasstroms wurde in Strömungsrichtung unmittelbar hinter der internen Aufheizschüttung auf 450°C eingestellt. Mit einem Verlassdruck von 2 bar über Atmosphärendruck wurde das Reduziergas über einen Zeitraum von 45 Minuten durch den Rohrreaktor geführt.

Während der letzten 5 Minuten wurde die Temperatur des Reduziergasstroms in Strömungsrichtung unmittelbar hinter der internen Aufheizschüttung auf 410°C eingestellt.

Dann wurde die Dehydrierung wieder in identischer Weise aufgenommen. Nach einer Betriebsdauer von ca. 1 h war der (quasi) stationäre Betriebszustand wieder erreicht. Unter den ursprünglichen Betriebsbedingungen wurde dabei der ursprüngliche Wert für G wieder erzielt. Das Deaktivierverhalten des regenerierten Katalysatorbetts entsprach demjenigen des frisch beschickten Katalysatorbetts. Während des gesamten Regenerierverfahrens lag die Temperatur im Rohrreaktor überall bei Werten $\leq$ 590°C. Den Verlauf des Gehaltes des den Rohrreaktor verlassenden Regeneriergases an $CO_2$ (1) und CO (2) in Vol.-% als Ordinate über die Regenerierzeit in Minuten als Abszisse weist Figur 4 aus (t = 10 min = Beginn der Zufuhr von Regeneriergas). Der Gesamtgehalt an Kohlenoxiden im den Rohrreaktor verlassenden Regeneriergas lag während der gesamten Regenerierdauer unterhalb von 2 Vol.-%.

II. Vergleichsbeispiel

Es wurde wie im Beispiel verfahren. Als Regeneriergasstrom wurde jedoch stets ein konstantes Gemisch aus 5 Vol.-% molekularem Sauerstoff und 95 Vol.-% molekularem Stickstoff verwendet. Die übrigen Randbedingungen (z.B. Betrag des Regeneriergasstroms, Verlassdruck, Temperatur des Regenerierungsstroms unmittelbar hinter der internen Aufheizschüttung) wurden identisch beibehalten. Im Anfangszeitraum der Regenerierung lag der Gesamtgehalt an Kohlenoxiden im den Rohrreaktor verlassenden Regeneriergas oberhalb von 5 Vol.-%. Nach der Wiederaufnahme der Dehydrierung wurde im stationären Betriebszustand unter den ursprünglichen Betriebsbedingungen der ursprüngliche Wert für G noch nicht wieder erreicht. Es wurde vielmehr nur ein G' (zum Betriebszeitpunkt $t_0$) von 16,3 mol-% erzielt. Die Deaktivierung des regenerierten Katalysatorbetts erfolgte rascher als im Fall des frisch beschickten Katalysatorbetts.

III. Vergleichsbeispiel

Es wurde wie im Beispiel verfahren. Innerhalb der ersten 60 Minuten des Regenerierverfahrens wurde jedoch ein Gasgemisch aus 5 Vol.-% molekularem Sauerstoff und 95 Vol.-% molekularem Stickstoff verwendet (im nachfolgenden Regenerierzeitraum entsprach die Regeneriergaszusammensetzung jeweils jener aus dem Beispiel). Die übrigen Randbedingungen (z.B. Betrag des Regeneriergasstroms, Verlassdruck, Temperatur des Regeneriergasstroms unmittelbar hinter der internen Aufheizschüttung) wurden identisch beibehalten. Im Anfangszeitraum der Regenerierung lag der Gesamtgehalt an Kohlenoxiden im den Rohrreaktor verlassenden Regeneriergas oberhalb von 5 Vol.-%.

Nach der Wiederaufnahme der Dehydrierung wurde im stationären Betriebszustand unter den ursprünglichen Betriebsbedingungen der ursprüngliche Wert für G noch nicht wieder erreicht. Es wurde vielmehr nur ein G* (zum Betriebszeitpunkt $t_0$) von 16,7 mol-% erzielt. Die Deaktivierung des regenerierten Katalysatorbetts erfolgte rascher als im Fall des frisch beschickten Katalysatorbetts.

US Provisional Patent Application No. 60/888366, eingereicht am 06.02.2007, ist eingefügt in die vorliegende Anmeldung durch Literaturhinweis.

Im Hinblick auf die obengenannten Lehren sind zahlreiche Änderungen und Abweichungen von der vorliegenden Erfindung möglich.

Man kann deshalb davon ausgehen, dass die Erfindung, im Rahmen der beigefügten Ansprüche, anders als hierin spezifisch beschrieben, ausgeführt werden kann.

**Patentansprüche**

1. Verfahren zur Regenerierung eines im Rahmen einer heterogen katalysierten partiellen Dehydrierung eines zu dehydrierenden Kohlenwasserstoffs zu einem dehydrierten Kohlenwasserstoff deaktivierten Katalysatorbetts, das über einen Zeitraum t das Durchleiten eines molekularen Sauerstoff und Inertgas, aber keinen Kohlenwasserstoff enthaltenden Regeneriergases mit erhöhter Temperatur durch das deaktivierte Katalysatorbett mit der Maßgabe umfasst, dass der Gesamtgehalt $G^A$ des durch das Katalysatorbett geleiteten Regeneriergases an Kohlenoxiden bei dessen Austritt aus dem Katalysatorbett im Verlauf der Regenerierung über den Zeitraum t wenigstens zeitweise größer ist, als der entsprechende Gehalt $G^E$, jeweils ausgedrückt in Vol.-% des Regeneriergasvolumens, desselben durch das Katalysatorbett geleiteten Regeneriergases bei dessen Eintritt in das Katalysatorbett und die Differenz $\Delta G = G^A - G^E$ bis zur Beendigung des Regenerierverfahrens einen Höchstwert $\Delta G^{max}$ durchläuft, **dadurch gekennzeichnet, dass**

　　a) 0,2 Vol.-% $\leq \Delta G^{max} \leq$ 5 Vol.-% und

　　b) der Gehalt des durch das Katalysatorbett zu leitenden Regeneriergases an molekularem Sauerstoff, ausgedrückt in Vol.-% des Regeneriergasvolumens, innerhalb des Zeitraums t bis zur Beendigung des Regenerierverfahrens wenigstens dreimal erhöht wird und die jeweilige Erhöhung wenigstens 2 Vol.-% beträgt.

2. Verfahren nach Anspruch 1, **dadurch gekennzeichnet, dass** 0,2 Vol.-% $\leq \Delta G^{max} \leq$ 4 Vol.-%.

3. Verfahren nach Anspruch 1, **dadurch gekennzeichnet, dass** 0,2 Vol.-% $\leq \Delta G^{max} \leq$ 3 Vol.-%.

4. Verfahren nach einem der Ansprüche 1 bis 3, **dadurch gekennzeichnet, dass** der Sauerstoffgehalt des durch das Katalysatorbett zu leitenden Regeneriergases innerhalb des Zeitraums t insgesamt um wenigstens 10 Vol.-% erhöht wird.

5. Verfahren nach einem der Ansprüche 1 bis 3, **dadurch gekennzeichnet, dass** der Sauerstoffgehalt des durch das Katalysatorbett zu leitenden Regeneriergases innerhalb des Zeitraums t insgesamt um wenigstens 14 Vol.-% erhöht wird.

6. Verfahren nach einem der Ansprüche 1 bis 5, **dadurch gekennzeichnet, dass** der Gehalt des durch das Katalysatorbett zu leitenden Regeneriergases an molekularem Sauerstoff bei Beginn des Verfahrens $\leq$ 5 Vol.-% beträgt.

7. Verfahren nach einem der Ansprüche 1 bis 5, **dadurch gekennzeichnet, dass** der Gehalt des durch das Katalysatorbett zu leitenden Regeneriergases an molekularem Sauerstoff bei Beginn des Verfahrens $\leq$ 3 Vol.-% beträgt.

8. Verfahren nach einem der Ansprüche 1 bis 7, **dadurch gekennzeichnet, dass** die im Zeitraum t im Katalysatorbett auftretende höchste Temperatur T, bezogen auf die im Rahmen der die Deaktivierung des Katalysatorbetts bedingenden heterogen katalysierten partiellen Dehydrierung im Katalysatorbett aufgetretene höchste Temperatur $T^{max}$ folgende Bedeutung erfüllt:

$$0,5 \bullet T^{max} \leq T \leq 1,5 \bullet T^{max}.$$

9. Verfahren nach einem der Ansprüche 1 bis 8, **dadurch gekennzeichnet, dass** die Belastung B des Katalysatorbetts mit Regeneriergas

$$1\ 000\ Nl/l\bullet h \leq B \leq 40\ 000\ Nl/l\bullet h$$

beträgt.

10. Verfahren nach einem der Ansprüche 1 bis 8, **dadurch gekennzeichnet, dass** die Belastung B des Katalysatorbetts mit Regerniergas

$$3\ 000\ Nl/l\bullet h \leq B \leq 20\ 000\ Nl/l\bullet h$$

beträgt.

11. Verfahren nach einem der Ansprüche 1 bis 10, **dadurch gekennzeichnet, dass** die Belastung B des Katalysatorbetts mit Regeneriergas innerhalb des Zeitraums t ein Maximum durchläuft.

12. Verfahren nach einem der Ansprüche 1 bis 11, **dadurch gekennzeichnet, dass** das Regeneriergas als Inertgas $N_2$ und/oder $H_2O$ enthält.

13. Verfahren nach einem der Ansprüche 1 bis 12, **dadurch gekennzeichnet, dass** man wenigstens eine Teilmenge des aus dem Katalysatorbett austretenden Regeneriergases im Kreis führt und als Bestandteil von frisch durch das Katalysatorbett zu führendem Regeneriergas wiederverwendet.

14. Verfahren nach Anspruch 13, **dadurch gekennzeichnet, dass** der Gehalt des frisch durch das Katalysatorbett zu führenden Regeneriergases an CO $\leq$ 3 Vol.-% beträgt.

15. Verfahren nach einem der Ansprüche 1 bis 14, **dadurch gekennzeichnet, dass** es bei einem Arbeitsdruck von 1 bis 10 bar oberhalb von Atmosphärendruck durchgeführt wird.

16. Verfahren nach einem der Ansprüche 1 bis 15, **dadurch gekennzeichnet, dass** der Unterschied $T^A - T^E$ zwischen der Temperatur des Regeneriergases beim Eintritt in das Katalysatorbett, $T^E$, und der Temperatur desselben Regeneriergases beim Austritt aus dem Katalysatorbett, $T^A$, $\leq 250°C$ beträgt.

17. Verfahren nach einem der Ansprüche 1 bis 16, **dadurch gekennzeichnet, dass** das Katalysatorbett ein Katalysatorfestbett ist.

18. Verfahren nach Anspruch 17, **dadurch gekennzeichnet, dass** das Schüttvolumen des Katalysatorfestbetts $\geq 50$ l und $\leq 10\,000$ m$^3$ beträgt.

19. Verfahren nach einem der Ansprüche 1 bis 18, **dadurch gekennzeichnet, dass** das Katalysatorbett Katalysatoren enthält, die aus wenigstens einem auf einem oxidischen Träger abgeschiedenen Metall bestehen.

20. Verfahren nach Anspruch 18, **dadurch gekennzeichnet, dass** das wenigstens eine Metall ein Metall aus der Gruppe bestehend aus Cu, Ag, Au, Zn, Cd, Hg, Ru, Rh, Pd, Os, Ir und Pt ist.

21. Verfahren nach einem der Ansprüche 1 bis 19, **dadurch gekennzeichnet, dass** das Katalysatorbett sich in einem adiabat gestalteten Reaktor befindet.

22. Verfahren zur Herstellung von Acrolein und/oder Acrylsäure durch heterogen katalysierte partielle Gasphasenoxidation von Propylen, umfassend als erste Reaktionsstufe eine heterogen katalysierte partielle Dehydrierung von Propan zu Propylen an einem Katalysatorbett, **dadurch gekennzeichnet, dass** das Katalysatorbett von Zeit zu Zeit nach einem Verfahren gemäß einem der Ansprüche 1 bis 21 regeneriert wird.

**Claims**

1. A process for regenerating a catalyst bed deactivated in the course of a heterogeneously catalyzed partial dehydrogenation of a hydrocarbon to be dehydrogenated to a dehydrogenated hydrocarbon, which comprises, over a period t, the passage of a regeneration gas comprising molecular oxygen and inert gas but no hydrocarbon at elevated temperature through the deactivated catalyst bed with the proviso that the total content $G^A$ of carbon oxides in the regeneration gas passed through the catalyst bed, when it exits from the catalyst bed, is at least temporarily greater in the course of the regeneration over the period t than the corresponding content $G^E$, in each case expressed in % by volume of the regeneration gas volume, of the same regeneration gas passed through the catalyst bed when it enters the catalyst bed, and the difference $\Delta G = G^A - G^E$ passes through a maximum value $\Delta G^{max}$ by the end of the regeneration process, wherein

   a) 0.2% by volume $\leq \Delta G^{max} \leq 5\%$ by volume and
   b) the content of molecular oxygen in the regeneration gas to be passed through the catalyst bed, expressed in % by volume of the regeneration gas volume, is increased at least three times during the period t up to the end of the regeneration process and each increase is at least 2% by volume.

2. The process according to claim 1, wherein 0.2% by volume $\leq \Delta G^{max} \leq 4\%$ by volume.

3. The process according to claim 1, wherein 0.2% by volume $\leq \Delta G^{max} \leq 3\%$ by volume.

4. The process according to any of claims 1 to 3, wherein the oxygen content of the regeneration gas to be passed through the catalyst bed is increased by at least 10% by volume in total within the period t.

5. The process according to any of claims 1 to 3, wherein the oxygen content of the regeneration gas to be passed through the catalyst bed is increased by at least 14% by volume in total within the period t.

6. The process according to any of claims 1 to 5, wherein the content of molecular oxygen in the regeneration gas to be passed through the catalyst bed on commencement of the process is $\leq 5\%$ by volume.

7. The process according to any of claims 1 to 5, wherein the content of molecular oxygen in the regeneration gas to be passed through the catalyst bed on commencement of the process is ≤ 3% by volume.

8. The process according to any of claims 1 to 7, wherein the highest temperature T occurring within the period t in the catalyst bed, based on the highest temperature $T^{max}$ which occurred in the catalyst bed in the course of the heterogeneously catalyzed partial dehydrogenation which causes the deactivation of the catalyst bed, fulfils the following condition:

$$0.5 \cdot T^{max} \leq T \leq 1.5 \cdot T^{max}.$$

9. The process according to any of claims 1 to 8, wherein the loading B of the catalyst bed with regeneration gas is

$$1000 \ l \ (STP)/l \cdot h \leq B \leq 40\ 000 \ l \ (STP)/l \cdot h.$$

10. The process according to any of claims 1 to 8, wherein the loading B of the catalyst bed with regeneration gas is

$$3000 \ l \ (STP)/l \cdot h \leq B \leq 20\ 000 \ l \ (STP)/l \cdot h.$$

11. The process according to any of claims 1 to 10, wherein the loading B of the catalyst bed with regeneration gas passes through a maximum within the period t.

12. The process according to any of claims 1 to 11, wherein the regeneration gas comprises $N_2$ and/or $H_2O$ as inert gas.

13. The process according to any of claims 1 to 12, wherein at least a portion of the regeneration gas exiting from the catalyst bed is circulated and reused as a constituent of regeneration gas to be conducted freshly through the catalyst bed.

14. The process according to claim 13, wherein the content of CO in the regeneration gas to be conducted freshly through the catalyst bed is ≤ 3% by volume.

15. The process according to any of claims 1 to 14, which is performed at a working pressure of from 1 to 10 bar above atmospheric pressure.

16. The process according to any of claims 1 to 15, wherein the difference $T^A$ - $T^E$ between the temperature of the regeneration gas on entry into the catalyst bed, $T^E$, and the temperature of the same regeneration gas on exit from the catalyst bed, $T^A$, is ≤ 250°C.

17. The process according to any of claims 1 to 16, wherein the catalyst bed is a fixed catalyst bed.

18. The process according to claim 17, wherein the bed volume of the fixed catalyst bed is ≥ 50 l and ≤ 10 000 m³.

19. The process according to any of claims 1 to 18, wherein the catalyst bed comprises catalysts which consist of at least one metal deposited on an oxidic support.

20. The process according to claim 18, wherein the at least one metal is a metal from the group consisting of Cu, Ag, Au, Zn, Cd, Hg, Ru, Rh, Pd, Os, Ir and Pt.

21. The process according to any of claims 1 to 19, wherein the catalyst bed is disposed in an adiabatic reactor.

22. A process for preparing acrolein and/or acrylic acid by heterogeneously catalyzed partial gas phase oxidation of propylene, comprising, as a first reaction stage, a heterogeneously catalyzed partial dehydrogenation of propane to propylene on a catalyst bed, which comprises regenerating the catalyst bed from time to time by a process according to any of claims 1 to 21.

**Revendications**

1. Procédé de régénération d'un lit catalytique désactivé dans le cadre d'une déshydrogénation partielle sous catalyse hétérogène d'un hydrocarbure à déshydrogéner en un hydrocarbure déshydrogéné, qui comprend le passage, pendant une durée t, d'un gaz de régénération contenant de l'oxygène moléculaire et un gaz inerte, mais pas d'hydrocarbure, à température élevée, au travers du lit catalytique désactivé, à condition que la teneur totale $G^A$ du gaz de régénération traversant le lit catalytique en oxydes de carbone lors de sa sortie du lit catalytique soit, au moins périodiquement au cours de la régénération pendant la durée t, supérieure à la teneur $G^E$ correspondante, à chaque fois exprimée en % en volume du volume du gaz de régénération, du même gaz de régénération traversant le lit catalytique lors de son entrée dans le lit catalytique, et la différence $\Delta G = G^A - G^E$ passe par une valeur maximale $\Delta G^{max}$ jusqu'à la fin du procédé de régénération, **caractérisé en ce que**

   a) 0,2 % en volume $\leq \Delta G^{max} \leq$ 5 % en volume et
   b) la teneur du gaz de régénération traversant le lit catalytique en oxygène moléculaire, exprimée en % en volume du volume du gaz de régénération, augmente au moins trois fois pendant la durée t jusqu'à la fin du procédé de régénération, et l'élévation correspondante est d'au moins 2 % en volume.

2. Procédé selon la revendication 1, **caractérisé en ce que** 0,2 % en volume $\leq \Delta G^{max} \leq$ 4 % en volume.

3. Procédé selon la revendication 1, **caractérisé en ce que** 0,2 % en volume $\leq \Delta G^{max} \leq$ 3 % en volume.

4. Procédé selon l'une quelconque des revendications 1 à 3, **caractérisé en ce que** la teneur en oxygène du gaz de régénération traversant le lit catalytique augmente au total d'au moins 10 % en volume pendant la durée t.

5. Procédé selon l'une quelconque des revendications 1 à 3, **caractérisé en ce que** la teneur en oxygène du gaz de régénération traversant le lit catalytique augmente au total d'au moins 14 % en volume pendant la durée t.

6. Procédé selon l'une quelconque des revendications 1 à 5, **caractérisé en ce que** la teneur du gaz de régénération traversant le lit catalytique en oxygène moléculaire au début du procédé est $\leq$ 5 % en volume.

7. Procédé selon l'une quelconque des revendications 1 à 5, **caractérisé en ce que** la teneur du gaz de régénération traversant le lit catalytique en oxygène moléculaire au début du procédé est $\leq$ 3 % en volume.

8. Procédé selon l'une quelconque des revendications 1 à 7, **caractérisé en ce que** la température T la plus élevée se produisant dans le lit catalytique pendant la durée t, par rapport à la température $T^{max}$ la plus élevée s'étant produite dans le lit catalytique dans le cadre de la déshydrogénation partielle sous catalyse hétérogène provoquant la désactivation du lit catalytique, satisfait la signification suivante :

$$0,5 \cdot T^{max} \leq T \leq 1,5 \cdot T^{max}.$$

9. Procédé selon l'une quelconque des revendications 1 à 8, **caractérisé en ce que** le chargement B du lit catalytique avec le gaz de régénération est de

$$1\ 000\ Nl/l \cdot h \leq B \leq 40\ 000\ Nl/l \cdot h.$$

10. Procédé selon l'une quelconque des revendications 1 à 8, **caractérisé en ce que** le chargement B du lit catalytique avec le gaz de régénération est de

$$3\ 000\ Nl/l \cdot h \leq B \leq 20\ 000\ Nl/l \cdot h.$$

11. Procédé selon l'une quelconque des revendications 1 à 10, **caractérisé en ce que** le chargement B du lit catalytique avec le gaz de régénération passe par un maximum pendant la durée t.

12. Procédé selon l'une quelconque des revendications 1 à 11, **caractérisé en ce que** le gaz de régénération contient

N$_2$ et/ou H$_2$O en tant que gaz inerte.

**13.** Procédé selon l'une quelconque des revendications 1 à 12, **caractérisé en ce qu'**au moins une partie du gaz de régénération sortant du lit catalytique est mise en circulation et réutilisée en tant que constituant d'un gaz de régénération frais à faire passer au travers du lit catalytique.

**14.** Procédé selon la revendication 13, **caractérisé en ce que** la teneur du gaz de régénération frais à faire passer au travers du lit catalytique en CO est ≤ 3 % en volume.

**15.** Procédé selon l'une quelconque des revendications 1 à 14, **caractérisé en ce qu'**il est réalisé à une pression de travail de 1 à 10 bar au-dessus de la pression atmosphérique.

**16.** Procédé selon l'une quelconque des revendications 1 à 15, **caractérisé en ce que** la différence T$^A$-T$^E$ entre la température du gaz de régénération lors de l'entrée dans le lit catalytique, T$^E$ et la température du même gaz de régénération lors de la sortie du lit catalytique, T$^A$, est ≤ 250 °C.

**17.** Procédé selon l'une quelconque des revendications 1 à 16, **caractérisé en ce que** le lit catalytique est un lit catalytique fixe.

**18.** Procédé selon la revendication 17, **caractérisé en ce que** le volume de garnissage du lit catalytique fixe est ≥ 50 l et ≤ 10 000 m$^3$.

**19.** Procédé selon l'une quelconque des revendications 1 à 18, **caractérisé en ce que** le lit catalytique contient des catalyseurs qui sont constitués d'au moins un métal déposé sur un support oxydique.

**20.** Procédé selon la revendication 18, **caractérisé en ce que** le ou les métaux sont un métal du groupe constitué par Cu, Ag, Au, Zn, Cd, Hg, Ru, Rh, Pd, Os, Ir et Pt.

**21.** Procédé selon l'une quelconque des revendications 1 à 19, **caractérisé en ce que** le lit catalytique se trouve dans un réacteur configuré de manière adiabatique.

**22.** Procédé de fabrication d'acroléine et/ou d'acide acrylique par oxydation partielle en phase gazeuse sous catalyse hétérogène de propylène, comprenant en tant que première étape de réaction une déshydrogénation partielle sous catalyse hétérogène de propane en propylène sur un lit catalytique, **caractérisé en ce que** le lit catalytique est régénéré de temps en temps par un procédé selon l'une quelconque des revendications 1 à 21.

Figur 1

Figur 2

Figur 3

Figur 4

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 03076370 A
- DE 102004032129 A
- EP 731077 A
- WO 0196271 A
- WO 0196270 A
- DE 10316039 A
- WO 03011804 A
- WO 0010961 A
- EP 799169 A
- DE 10245585 A
- DE 102005061626
- DE 102006017623
- DE 102006035718
- DE 102006024901
- WO 0196008 A
- DE 102006029790 A
- DE 10351269 A
- DE 10350812 A
- DE 10350822 A
- DE 10028582 A
- DE 102006035718 A
- DE 102005013039 A
- DE 10219879 A
- DE 10131297 A
- WO 9946039 A
- US 4788371 A
- EP 705136 A
- WO 9929420 A
- US 4220091 A
- US 5430220 A
- US 5877369 A
- EP 117146 A
- DE 19937196 A
- DE 19937105 A
- US 3670044 A
- US 6566573 A
- WO 0183405 A
- WO 9429021 A
- CN 1073893 A
- WO 9736849 A
- DE 19753817 A
- US 3862256 A
- US 3887631 A
- DE 19530454 A
- US 4341664 A
- US 5086032 A
- US 4255284 A
- DE 19837520 A
- DE 19837517 A
- DE 19837519 A
- DE 19837518 A
- EP 938463 A
- EP 167109 A
- DE 19838312 A
- DE 102005044916
- DE 19937170 A
- DE 19937107 A
- DE 1020060017623 A
- DE 102006015235 A
- DE 102005061626 A
- EP 1109763 A
- US 3308181 A
- US 4886928 A
- US 5430209 A
- US 5530171 A
- US 5527979 A
- US 5563314 A
- DE 10211275 A
- DE 102006017623 A
- EP 700893 A
- EP 700714 A
- DE 102005022798 A
- DE 10246119 A
- DE 102005049699 A
- DE 102005010111 A
- DE 102005009891 A
- US 3161670 A
- DE 3313573 A
- DE 10219686 A
- DE 102005009885 A
- DE 102005052923 A
- DE 102005057197 A
- DE 2213573 A
- DE 102005013039
- US 60888366 B

**In der Beschreibung aufgeführte Nicht-Patentliteratur**

- *Catalysis Letters,* 1994, vol. 23, 103-106
- **W. ZHANG.** *Gaodeng Xuexiao Huaxue Xuebao,* 1993, vol. 14, 566

- **Z. HUANG.** *Shiyon Huagong,* 1992, vol. 21, 592
- *J. of Catalysis,* 1997, vol. 167, 560-569
- *J. of Catalysis,* 1997, vol. 167, 550-559
- *Topics in Catalysis,* 1996, vol. 3, 265-275
- *Catalysis Letters,* 1991, vol. 10, 181-192
- *Ind. Eng. Chem. Res.,* 1996, vol. 35, 14-18
- *Applied Catalysis A: General,* 1993, vol. 100, 111-130
- *J. of Catalysis,* 1994, vol. 148, 56-67
- New Developments in Selective Oxidation II. Elsevier Science B.V, 1994, 305-313
- 3rd World Congress on Oxidation Catalysis. Elsevier Science B.V, 1997, 375ff
- **Z. HUANG.** *Shiyou Huagong,* 1992, vol. 21, 592